Europäisches Patentamt
European Patent Office
Office européen des brevets

(19)

(11) Publication number: 0 060 077 A1

# (12) EUROPEAN PATENT APPLICATION

(21) Application number: 82301061.6

(22) Date of filing: 02.03.82

(51) Int. Cl.³: C 07 D 487/04
A 61 K 31/40
//C07D205/08, C07F9/65, C07D265/16, C07D498/04, C07C31/27, C07C31/36, C07C117/00, (C07D487/04, 209/00, 205/00)

(30) Priority: 04.03.81 GB 8106762

(43) Date of publication of application: 15.09.82 Bulletin 82/37

(84) Designated Contracting States: BE CH DE FR GB IT LI NL SE

(71) Applicant: BEECHAM GROUP PLC
Beecham House Great West Road
Brentford Middlesex(GB)

(72) Inventor: Smale, Terence Charles
28 St. Leonards Road
Epsom Downs Epsom Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Beta-lactam antibiotics, their preparation and use.**

(57) The present invention provides a compound of the formula (I):

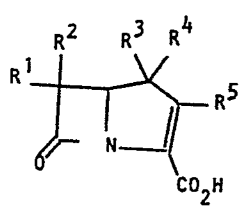

(I)

or a pharmaceutically acceptable salt or an ester thereof wherein $R^1$ and $R^2$ each represent a hydrogen atom or an organic group bonded *via* a carbon atom to the carbapenem ring, $R^3$ and $R^4$ each represent an organic group bonded *via* a carbon atom to the carbapenem ring, or $R^3$ and $R^4$ are joined so as to form together with the carbon atom to which they are attached an optionally substituted $C_{3-7}$ cycloalkyl or optionally substituted heterocyclyl ring, and $R^5$ is a hydrogen atom, a group SH, or a group $S(O)_nR^6$ wherein n is zero or one and $R^6$ is an organic group bonded *via* a carbon atom to the sulphur atom; excluding benzyl 4,4-dimethyl-7-oxo-1-azabicyclo[3.2.0.]hept-2-ene-2-carboxylate.

These compounds are useful as antibacterial agents.

EP 0 060 077 A1

Croydon Printing Company Ltd

## β-Lactam Antibiotics, their Preparation and Use

This invention relates to novel carbapenem derivatives and in particular to 4,4-disubstituted 7-oxo-1-azabicyclo[3.2.0]heptene derivatives. This invention further relates to processes for their preparation and to compositions containing them. These derivatives are of use in the treatment of bacterial infection.

The present invention provides a compound of the formula (I):

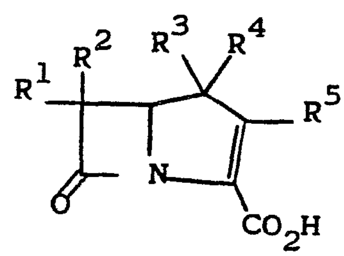

(I)

or a pharmaceutically acceptable salt or a pharmaceutically acceptable ester thereof wherein $R^1$ and $R^2$ each represent a hydrogen atom or an organic group bonded via a carbon atom to the azabicycloheptene ring, $R^3$ and $R^4$ each represent an organic group bonded via a carbon atom to the azabicycloheptene ring, or $R^3$ and $R^4$ are joined so as to form together with the carbon atom to which they are attached an optionally substituted $C_{3-7}$ cycloalkyl or optionally substituted heterocyclyl ring, and $R^5$ is a hydrogen atom, a group SH, or a group

$S(O)_nR^6$ wherein n is zero or one and $R^6$ is an organic group bonded via a carbon atom to the sulphur atom; excluding benzyl 4,4-dimethyl-7-oxo-1-azabicyclo[3.2.0] hept-2-ene-2-carboxylate.

Suitably $R^5$ is a hydrogen atom. Suitably $R^5$ is a group SH. More suitably $R^5$ is a group $-S(O)_nR^6$ wherein in one aspect n is one, and in another aspect n is zero.

The group $R^6$ in the compound of the formula (I) may be $C_{1-6}$ alkyl, $C_{3-7}$ cycloalkyl, $C_{3-7}$ cycloalkyl $(C_{1-6})$ alkyl, $C_{2-6}$ alkenyl, $C_{2-6}$ alkynyl, aryl $(C_{1-6})$ alkyl, $C_{1-6}$ alkanoyl, aryl $(C_{1-6})$ alkanoyl, arylcarbonyl, heteroarylcarbonyl, aryl, heterocyclyl, heterocyclyl $(C_{1-6})$ alkyl, heteroaryl $(C_{1-6})$ alkyl or heteroaryl group, any of such groups being optionally substituted. Suitably the hetero atom or hetero atoms in the above named heteroaryl and/or heterocyclyl moieties are selected from 1 to 4 oxygen, nitrogen or sulphur atoms, and the heteroaryl and/or heterocyclyl moieties comprise of up to 7 ring atoms, preferably 5 or 6 ring atoms.

Suitable optional substituents for the group $R^6$ include $C_{1-6}$ alkyl, amino, $C_{1-6}$ alkanoylamino, mono, di- and tri- $(C_{1-6})$ alkylamino, hydroxy, $C_{1-6}$ alkoxy, mercapto, $C_{1-6}$ alkylthio, heteroarylthio, arylthio, sulphamoyl, carbamoyl, amidino, guanidino, nitro, chloro, bromo, fluoro, carboxy and salts and esters thereof, $C_{1-6}$ alkanoyloxy, arylcarbonyl and heteroarylcarbonyl.

Suitably $R^6$ is $C_{1-6}$alkyl, $C_{2-6}$alkenyl, aryl such as phenyl, aralkyl wherein the aryl moiety is preferably phenyl and the alkyl part has 1 to 6 carbon atoms, for example benzyl, phenethyl; heterocyclyl or heterocyclylalkyl wherein the alkyl part has 1 to 3 carbon atoms and the heterocyclic ring comprises 4 to 7 atoms, preferably 5 to 6, up to 4 of which may be selected from oxygen, sulphur and nitrogen, such as pyridyl, furyl, thienyl, pyrimidinyl, imidazolyl, triazinyl and tetrazolyl, and more suitably furyl, thienyl, pyrimidinyl, imidazolyl and triazinyl.

Preferably $R^6$ is $C_{1-6}$alkyl for example methyl, ethyl or propyl, optionally substituted by amino, $C_{1-6}$ alkanoylamino, carboxy, mono- and di- alkylamino, hydroxy, amidino or $C_{1-6}$ alkoxy. Of these it is preferred that $R^6$ is ethyl substituted by $C_{1-6}$ alkanoylamino, for example $R^6$ may be acetamidoethyl.

Preferably also $R^6$ is optionally substituted $C_{2-6}$ alkenyl, and in particular optionally substituted vinyl wherein the substituents are selected from those described hereinbefore, in particular carbamoyl, carboxy and salts and esters thereof, and $C_{1-6}$ alkanoylamino. Suitable carbamoyl groups include mono- and di-$(C_{1-6})$alkyl carbamoyl, phenylcarbamoyl and $NH_2CO$-. Suitably the carboxy group is salified, for example by sodium or potassium, or is esterified with a $C_{1-6}$ alkyl ester such as methyl or ethyl, or with an amino $C_{1-6}$ alkyl ester such as aminoethyl. Suitable $C_{1-6}$ alkanoylamino groups include acetamido and propionamido, in particular acetamido is preferred.

In a further preferred embodiment $R^6$ is an optionally substituted phenyl, pyrimidinyl or pyridyl group, in particular optionally substituted pyrimidinyl is preferred. Suitable substituents for the pyrimidinyl ring include $C_{1-6}$ alkyl such as methyl, $C_{1-6}$ alkoxy, nitro, halo, amino, $C_{1-6}$ alkanoylamino and hydrogen.

Preferably in the compounds of the formula (I) either $R^1$ or $R^2$ is a hydrogen atom. In an alternative aspect both $R^1$ and $R^2$ are hydrogen atoms.

When the group $R^1$ or $R^2$ represents an organic group, it may be selected from $C_{1-10}$ alkyl, $C_{2-10}$ alkenyl, $C_{2-10}$ alkynyl, $C_{3-7}$ cycloalkyl ($C_{1-6}$) alkyl, $C_{3-7}$ cycloalkyl, aryl, aryl ($C_{1-6}$) alkyl, heterocyclyl, heterocyclyl ($C_{1-6}$) alkyl, heteroaryl, heteroaryl ($C_{1-6}$) alkyl, $C_{1-6}$ alkanoyl, aryl $C_{1-6}$ alkanoyl, arylcarbonyl and heteroarylcarbonyl, any of the above groups being optionally substituted. Suitably the hetero atom or hetero atoms in the above named heteroaryl and/or heterocyclyl moieties are selected from 1 to 4 oxygen, nitrogen or sulphur atoms. Suitable optional substituents

include $C_{1-6}$ alkyl, amino, $C_{1-6}$ alkanoylamino, mono, di- and tri- $(C_{1-6})$ alkylamino, hydroxy, $C_{1-6}$ alkoxy, mercapto, $C_{1-6}$ alkylthio, heteroarylthio, arylthio, sulphamoyl, carbamoyl, amidino, guanidino, nitro, chloro, bromo, fluoro, carboxy and salts and esters thereof, $C_{1-6}$ alkanoyloxy, arylcarbonyl and heteroarylcarbonyl.

More suitably one of $R^1$ and $R^2$ is a hydrogen atom and the other is selected from α-sulphonatooxyethyl or a group $CR^7R^8R^9$ wherein $R^7$ is a hydrogen atom or a hydroxy group, $R^8$ is a hydrogen atom or a $C_{1-6}$ alkyl group; and $R^9$ is a hydrogen atom, a $C_{1-6}$ alkyl group, a benzyl group, a phenyl group, or is joined to $R^8$ to form together with the carbon atom to which they are joined a carbocyclic ring of 5 to 7 carbon atoms.

Suitably $R^7$ is a hydrogen atom or a hydroxy group. Suitably $R^9$ is a hydrogen atom or a methyl, ethyl, n-propyl or phenyl group. Suitably $R^8$ is a hydrogen atom or a methyl, ethyl or n-propyl group. Favourably $R^8$ is a hydrogen atom or methyl group. Favourably $R^9$ is a hydrogen atom or methyl group.

Preferably the $CR^7R^8R^9$ moiety is a $-C(CH_3)_2OH$, $-CH(CH_3)OH$, $-CH(C_2H_5)OH$, $-CH_2CH_3$ or $-CH(CH_3)_2$ group, of

these the $-CH(CH_3)OH$ group is most favoured.

It is to be realised that the compounds of the formula (I) wherein $R^7R^8R^9$ have different values may exist in either the 8R or 8S form (the C-8 carbon atom being that adjacent to the carbon atom of the carbapenem ring). If desired these compounds may be presented as the 8R or the 8S isomer or as a mixture thereof.

Suitably $R^3$ and $R^4$ represent hydrocarbon groups, in particular having from 1 to 20 carbon atoms, especially 1 to 10 carbon atoms.

For example the groups $R^3$ and $R^4$ may be selected from $C_{1-6}$ alkyl, $C_{3-7}$ cycloalkyl, $C_{3-7}$ cycloalkyl $(C_{1-6})$ alkyl, aryl $(C_{1-6})$alkyl, heteroaryl $(C_{1-6})$alkyl, heterocyclyl $(C_{1-6})$alkyl, heterocyclyl, aryl and heteroaryl. Suitably the hetero atom or heteroatoms in the above named heteroaryl and/or heterocyclyl moieties are selected from 1 to 4 oxygen, nitrogen or sulphur atoms.

Suitably also $R^3$ and $R^4$ are joined so as to form together with the carbon atom to which they are attached an optionally substituted $C_{3-7}$ cycloalkyl or optionally substituted heterocyclyl ring; for example cyclopropyl, cyclobutyl, cyclopentyl or cyclohexyl. Of these cyclopropyl is preferred.

More suitably groups $R^3$ and $R^4$ may be selected from $C_{1-6}$ alkyl such as methyl, ethyl or n-propyl; aryl $(C_{1-6})$ alkyl such as benzyl or phenethyl; or $C_{3-7}$ cycloalkyl such as cyclohexyl.

Preferably $R^3$ is $C_{1-6}$ alkyl, in particular methyl and $R^4$ is $C_{1-6}$ alkyl, in particular methyl.

A preferred sub-group of compounds is that of the formula (II):

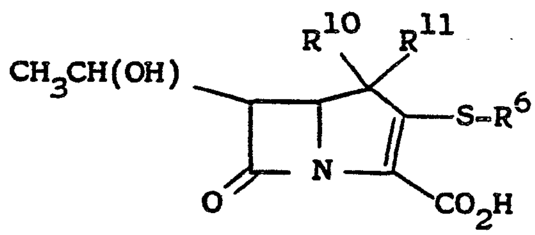

(II)

or a pharmaceutically acceptable salt or an ester thereof wherein $R^6$ is as hereinbefore defined and $R^{10}$ and $R^{11}$ are each $C_{1-6}$ alkyl. Preferably $R^{10}$ and $R^{11}$ are each methyl.

The compound of the formula (I) wherein one of $R^1$ and $R^2$ (but not both) is hydrogen may have the cis- or trans- configuration about the β-lactam ring, or they may be provided as mixtures thereof.

The compounds of the formulae (I) - (II) may have 5S or 5R stereochemistry or may be provided as mixtures thereof. It is believed that the more active isomer is that which exemplified in relation for formula (I) has the configuration shown in formula (III):

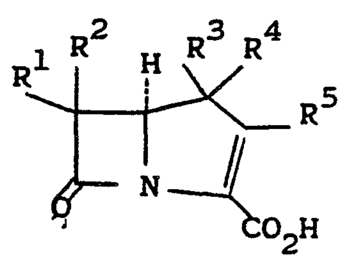

(III)

Suitable esters of the compounds of the formulae (I) - (III) include those cleavable by biological methods such as <u>in-vivo</u> hydrolysis, and those cleavable by chemical methods such as hydrogenolysis, hydrolysis, electrolysis or photolysis.

Suitably the carboxylic acid is esterified by a group of the sub-formula (a), (b), (c), (d), (e) or (f):

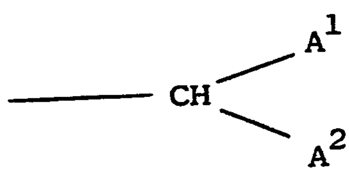
(a)

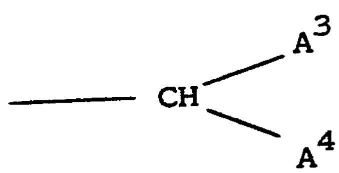
(b)

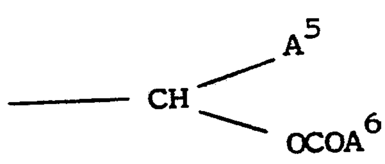
(c)

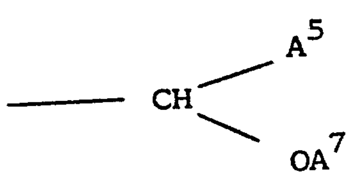
(d)

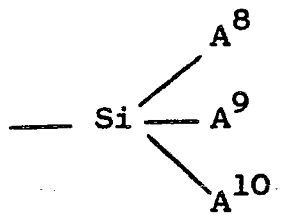
(e)

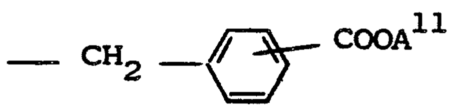
(f)

wherein $A^1$ is a hydrogen atom, $C_{1-6}$ alkanoyl or an alkyl, alkenyl or alkynyl group of up to 3 carbon atoms; $A^2$ is a hydrogen atom or a methyl group; $A^3$ is a phenyl group or a phenyl group substituted by a fluorine, chlorine or bromine atom or a nitro, methyl or methoxy group; $A^4$ is a hydrogen atom or a phenyl group or a phenyl group substituted by a fluorine, chlorine or bromine atom or a nitro, methyl or methoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacylmethyl or bromophenacylmethyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl

or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable _in-vivo_ hydrolysable esters are those esters which hydrolyse in the human body to produce the parent acid or its salt. Such esters may be identified by administration to a test animal such

as a rat or mouse by intravenous administration and thereafter examining the test animal's body fluids for the presence of the compound of the formula (I) or its salt.

Suitable esters of this type include those of sub-formula (c) as hereinbefore defined.

Suitable pharmaceutically acceptable salts include those of the alkali and alkaline earth metals, of these the sodium and potassium salts are preferred. These pharmaceutically acceptable salts may be formed at the C-2 carboxy and/or at the C-6 sulphonato-oxyethyl moiety (if present). Thus compounds of the formula (I) wherein $R^1$ contains a $OSO_3H$ group or pharmaceutically acceptable salt thereof, may be in the form of a di-salt such as the di-sodium or di-potassium salt, or may be in the form of a mono-salt of an <u>in-vivo</u> hydrolysable ester, or may be in the form of a mono-salt of an acid or may be in the form of a di-acid.

Non-pharmaceutically acceptable salts of the compounds of the formula (I) are also of use as they may be converted to the compound of the formula (I) or a pharmaceutically acceptable salt or an ester thereof.

In a further aspect of this invention there is provided a pharmaceutical composition which comprises a

compound of the formula (I) or pharmaceutically acceptable salt or an ester thereof and a pharmaceutically acceptable carrier. Preferably any such ester is in-vivo hydrolysable.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit-dose composition adapted for administration by injection.

Unit dose forms according to this invention will normally contain from 50 to 500 mgs of a compound of this invention, for example about 62.5, 100, 125, 150, 200, 250 or 300 mgs. Such compositions may be administered from 1 to 6 times a day or more conveniently 2, 3 or 4 times a day so that the total daily dose for a 70 kg adult is about 200 to 2000 mgs, for example about 400, 600, 750, 1000 or 1500 mgs.

The compositions of this invention may be used to treat bacterial infection, in animals such as mammals including humans, for example infections of the respiratory tract,

urinary tract or soft tissues, or mastitis in cattle.

A preferred feature of the compounds of this invention is that they tend to show good stability in human tissue.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth, potato starch or polyvinylpolypyrrolidone, magnesium stearate or sodium lauryl sulphate.

Orally administrable forms of the compositions of this invention are most suitably in the form of unit-dose units such as tablets or capsules.

The present invention also provides synergistic pharmaceutical compositions which comprise a pharmaceutical composition as hereinbefore described which also contains a penicillin or a cephalosporin.

Suitable penicillins for inclusion in the compositions of this invention include benzyl penicillin, phenoxymethyl-penicillin, ampicillin or a pro-drug therefor amoxycillin or a pro-drug therefor, carbenicillin or a pro-drug therefor, ticarcillin or a pro-drug therefor, suncillin, sulbenicillin, azlocillin or mezlocillin.

Particularly suitable penicillins for inclusion in orally administrable compositions of this invention

include ampicillin and its orally administrable pro-drugs, amoxycillin and its orally administrable pro-drugs and orally administrable pro-drugs of carbenicillin. Thus particularly suitable penicillins include ampicillin anhydrate, ampicillin trihydrate, sodium ampicillin, talampicillin hydrochloride, pivampicillin hydrochloride and bacampicillin hydrochloride; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion in the orally administrable compositions of this invention is ampicillin trihydrate.

Particularly suitable penicillins for inclusion in injectably administrable compositions of this invention include injectable salts such as the sodium salt of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include

cephaloridine, cephalexin, cephradine, cefazolin and cephalothin.

A particularly suitable cephalosporin for inclusion in the orally administrable compositions of this invention is cephalexin.

Particularly suitable cephalosporins for inclusion in the injectably administrable compositions of this invention include cephaloridine, cefazolin and cephradine, generally as their pharmaceutically acceptable salt.

The weight ratio between compound of this invention and penicillin or cephalosporin is generally from 10:1 to 1:10, more usually from 5:1 to 1:5 and normally from 3:1 to 1:3.

The penicillin or cephalosporin is generally utilised in its conventionally administered amount.

In another aspect the present invention provides a process for the preparation of a compound of the formula (I) or pharmaceutically acceptable salt or an ester thereof, wherein $R^5$ is a group $S(O)_nR^6$ which process comprises the reaction of a compound of the formula (IV):

(IV) 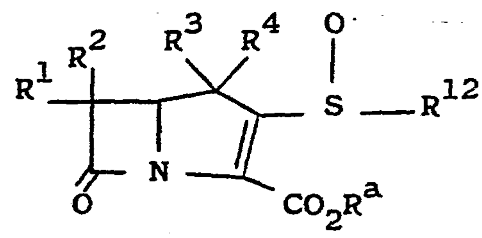

wherein $R^1$, $R^2$, $R^3$ and $R^4$ are as defined in relation to formula (I), $R^a$ is a carboxy-blocking group and $R^{12}$ is a $C_{1-6}$ alkyl, $C_{3-7}$ cycloalkyl, $C_{3-7}$ cycloalkyl $(C_{1-6})$ alkyl, aryl $(C_{1-6})$ alkyl, heterocyclyl $(C_{1-6})$ alkyl, heteroaryl $(C_{1-6})$ alkyl, heterocyclyl, heteroaryl, aryl, $C_{2-6}$ alkenyl or $C_{2-6}$ alkynyl group, any of such groups being optionally substituted; with a compound of the formula (V):

$$R^6 —— SH \qquad (V)$$

or reactive derivative thereof wherein $R^6$ is as defined in relation to formula (I); and thereafter if necessary:

i) removing any carboxy-blocking group,

ii) converting the product into a pharmaceutically acceptable salt or an ester,

iii) oxidising the sulphur atom to afford a sulphoxide.

This reaction may be performed in any solvent that is substantially inert during the reaction, for example tetrahydrofuran, dimethylformamide, dioxan, hexamethyl phosphoramide, dimethoxyethane or dimethoxydiethyl ether. Of these solvents dimethylformamide is preferred. Alternatively we have found it useful to use a phase transfer catalyst. Particularly suitable phase transfer catalysts include tetra-n-butyl ammonium bromide, cetylbromide and cetyltriethylammonium chloride; suitable solvents include halogenated water-immiscible solvents such as chloroform in the presence of water. The reaction is normally performed at ambient or a depressed temperature, for example 20°C to -70°C, and preferably between 0°C and -50°C. However when using a phase transfer catalyst it is preferably to conduct the reaction between 0°C and ambient temperature. When the thiol of the formula (V) is used the reaction is normally carried out in the presence of a base. Examples of such bases include sodium hydride, sodium hydroxide, sodium alkoxide such as the methoxide, ethoxide or butoxide, sodium amide, potassium hydroxide such as the methoxide ethoxide or butoxide, potassium amide, and trialkylamines such as triethylamine and tri-n-propylamine. Of these triethylamine is preferred. Preferably the base is present in an amount of at least 0.9 equivalents, more preferably between 1.0 and 1.2 equivalents per mole of the thiol compound. Instead of using a base in the reaction a reactive derivative of the thiol may be used, preferably the reactive derivative is a salt of the thiol, in particular an alkali metal salt such as sodium or potassium. The amount of thiol compound of the formula (V) or reactive derivative thereof is generally between

1.0 and 1.5 moles per mole equivalent of the compound of the formula (IV).

Suitable carboxyl-blocking derivatives for the group $-CO_2R^a$ in formulae (IV) include salts, esters, and anhydride derivatives of the carboxylic acid. The derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, and tertiary amine salts.

Suitable ester-forming carboxyl-blocking groups are those which may be removed under conventional conditions. Such groups for $R^a$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, acetonyl t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N{=}CHR^o$ where $R^o$ is aryl or heterocyclic, or an in-vivo hydrolysable ester.

The carboxyl group may be regenerated from any of the above esters by usual methods appropriate to the

particular $R^a$ group, for example, acid - and base - catalysed hydrolysis, or by enzymically - catalysed hydrolysis, or by hydrogenation. The hydrolysis must of course be carried out under conditions to which the groups on the rest of the molecule are stable.

When it is desired to produce a compound of formula (I) in the form of a free acid or salt by the process of this invention, a compound of formula (IV) is generally employed wherein $R^a$ is a carboxyl-blocking group. For the preparation of a compound of formula (I) in the form of a pharmaceutically acceptable ester, it is convenient to employ a compound of formula (IV) wherein $R^a$ represents the desired ester group.

Preferably the process of this invention is performed on a compound of the formula (IV) wherein $R^a$ is an ester-forming group.

Conversion of a compound of the formula (I) wherein n is zero to a compound of the formula (I) wherein n is one, may performed by use of an oxidising agent. Suitable oxidising agents include perbenzoic acid, hydrogen peroxide, selenium dioxide and sodium metaperiodate. Substituted perbenzoic acids such as m-chloroperbenzoic acid are preferred. The oxidation is conveniently performed in a substantially inert solvent such as dichloromethane, chloroform or carbon tetrachloride at an ambient or depressed temperature, preferably between -30°C to +20°C.

In another aspect the present invention provides a process for the preparation of a compound of the formula (VI):

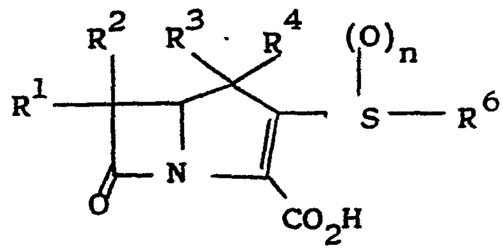

(VI)

or salt or ester thereof, wherein $R^1$, $R^2$, $R^3$, $R^4$, $R^6$ and n are as defined in relation to formula (I); which process comprises the elimination of the elements of HX from a compound of the formula (VII):

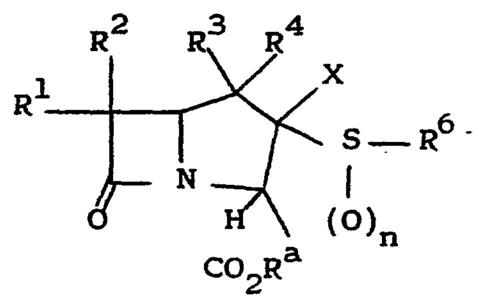

(VII)

wherein $R^1$, $R^2$, $R^3$, $R^4$, $R^6$ and n are as defined in relation to formula (VI), X is a halo group and $R^a$ is a carboxy-blocking group; and thereafter if necessary:

i) removing any carboxy-blocking group,

ii) converting the product into a pharmaceutically acceptable salt or an ester,

iii) oxidising the sulphur atom to afford a sulphoxide.

Preferably $R^6$ in the compounds of the formulae (VI) and (VII) is $C_{1-6}$ alkyl, $C_{3-7}$ cycloalkyl, $C_{3-7}$ cycloalkyl ($C_{1-6}$) alkyl, heterocyclyl, aryl ($C_{1-6}$) alkyl, heteroaryl ($C_{1-6}$) alkyl, heteroaryl, aryl or heterocyclyl ($C_{1-6}$) alkyl.

Suitably the elements HX are eliminated by the use of a base of low nucleophilicity, for example tertiary cyclic and acyclic amines such as N-methyl-moropholine and triethylamine, tertiary bicyclic diazoalkenes such as 1,5-diazabicyclo[4.3.0.]non-5-ene and 1,8-diazabicyclo [5.4.0.]undec-7-ene, and alkali metal bases of low nucleophilicity such as sodium thiophenoxide.

Suitably X is chloro, bromo or iodo.

The reaction is suitably performed in an organic solvent. The reaction may be performed at any non-extreme temperature, for example $-50^{o}C$ to $+50^{o}C$, preferably $-20^{o}C$ to $+ 40^{o}C$ and most conveniently at ambient temperature. It is preferred to perform the reaction under an inert atmosphere, for example using argon or nitrogen. For the compounds of the formula (VII) wherein n is one preferred solvents include ethyl acetate, N,N-dimethylformamide or dimethylsulphoxide. For the compounds of the formula (VII) wherein n is zero preferred solvents include dichloromethane and benzene.

The compounds of the formula (VII) are novel and as such form part of this invention.

The compounds of the formula (VII) wherein n is one may be prepared by the reaction of a compound of the formula (VIII):

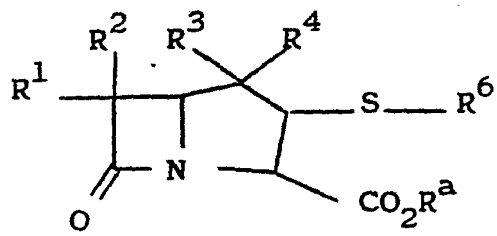

(VIII)

wherein $R^1$, $R^2$, $R^3$, $R^4$, $R^6$ and $R^a$ are as defined in relation to formula (VII), with an oxidising agent, and thereafter reacting the sulphoxide so produced with a source of electrophilic halogen. Thus this reaction is essentially a two-step oxidative halogenation, but the whole process may be carried out without the need to isolate any intermediates.

Suitable oxidising agents include per-acids and in particular perbenzoic acid and m-chloroperbenzoic acid. Sources of electrophilic halogen include N-haloamides and N-haloimides, for example N-halosuccinimides, sulphuryl halides and elemental halogens.

We have found that both oxidation and halogenation can be conveniently carried out using approximately 2 molar proportions of iodobenzene dichloride and at least 1 molar proportion of water in an organic solvent in the presence of 3 molar proportions of an organic base. Organic bases which are suitable for use in the iodobenzene dichloride process include tertiary aliphatic

amines such as triethylamine and aromatic bases such as pyridine.

The reaction is carried out in a organic solvent the choice of which is not critical to the success of these reactions. We have found that aprotic solvents such as halogenated hydrocarbons, benzene and toluene are most convenient.

The reactions are best carried out at moderate to low temperatures, i.e. not greater than 30°C and not less than -20°C, 0°C being convenient. Although it is not essential, the reaction may be usefully carried out under an inert atmosphere such as nitrogen or argon.

The compounds of the formula (VII) wherein n iz zero may be prepared by reacting a compound of the formula (VIII) with a halogenating agent. Preferably one equivalent of a halogenating agent is used, in the presence of base. Preferably iodobenzene dichloride is the halogenating agent.

The compound of the formula (VIII) and its sulphoxide are novel and as such form part of this invention.

The compound of the formula (VIII) may be prepared by the addition of a thiol $R^6SH$ to a compound of the formula (IX):

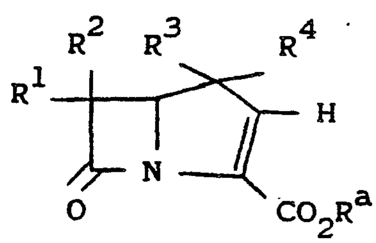

(IX)

wherein $R^1$, $R^2$, $R^3$, $R^4$ and $R^a$ are as defined in relation to formula (VIII).

The addition of the thiol usually takes place in a polar organic solvent such as dimethylformamide at a non-extreme temperature, for example -20°C to +40°C and most conveniently at ambient. Normally the addition reaction is carried out in the presence of a base such as a carbonate or bicarbonate such as those of the alkali or alkaline earth metals for example potassium carbonate.

The compound of the formula (IX) may be prepared by the ring closing elimination of the elements of $R^{14}R^{15}R^{16}P{=}O$ from an ester of a compound of the formula (X):

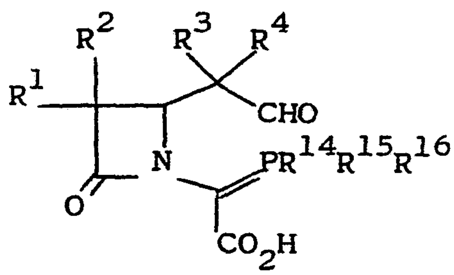

(X)

wherein $R^1$, $R^2$, $R^3$ and $R^4$ are as defined in relation to formula (IX), and $R^{14}$, $R^{15}$ and $R^{16}$ are independently phenyl or methoxyphenyl, and thereafter if desired converting the ester group to an alternative carboxy-blocking group. The solvent used is an inert organic solvent such as ethyl acetate. Ring closure tends to occur spontaneously so it is sometimes convenient to prepare and ring-close the phosphorane aldehyde in situ.

In an alternative aspect the present invention provides a process for the preparation of a compound of the formula (XI):

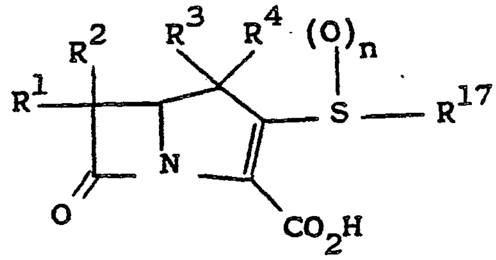

(XI)

or salt or ester thereof, wherein $R^1$, $R^2$, $R^3$, $R^4$ and n are as defined in relation to formula (I) and $R^{17}$ is an organic radical bonded to the sulphur atom via the carbon atom, said radical having an unsaturated moiety located adjacent to the sulphur atom; which process comprises the elimination of the elements of $R^{14}R^{15}R^{16}P{=}O$ from an ester of a compound of the formula (XII):

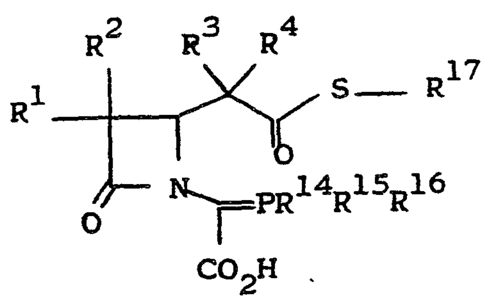

(XII)

wherein $R^1$, $R^2$, $R^3$, $R^4$, $R^{14}$, $R^{15}$, $R^{16}$ and $R^{17}$ are as hereinbefore defined, and thereafter if desired:

i) converting the ester to an acid, salt or another ester,

ii) oxidising the sulphur atom to a SO group.

Suitably this process is performed by heating the ester of the compound of the formula (XII) in an inert solvent, for example temperatures of 90° to 120°C and more suitably 100 to 110°C may be employed in a solvent such as toluene, preferably under substantially anhydrous conditions.

Preferably $R^{17}$ is a $C_{2-6}$ alkenyl, aryl or heteroaryl group, any of such groups being optionally substituted.

Compounds of the formulae (X) and (XII) may be prepared by the methods of European Patent Application Publication Number 0008888, 0008514 and 0002564; which methods are represented in Schemes 1 and 2.

SCHEME 1

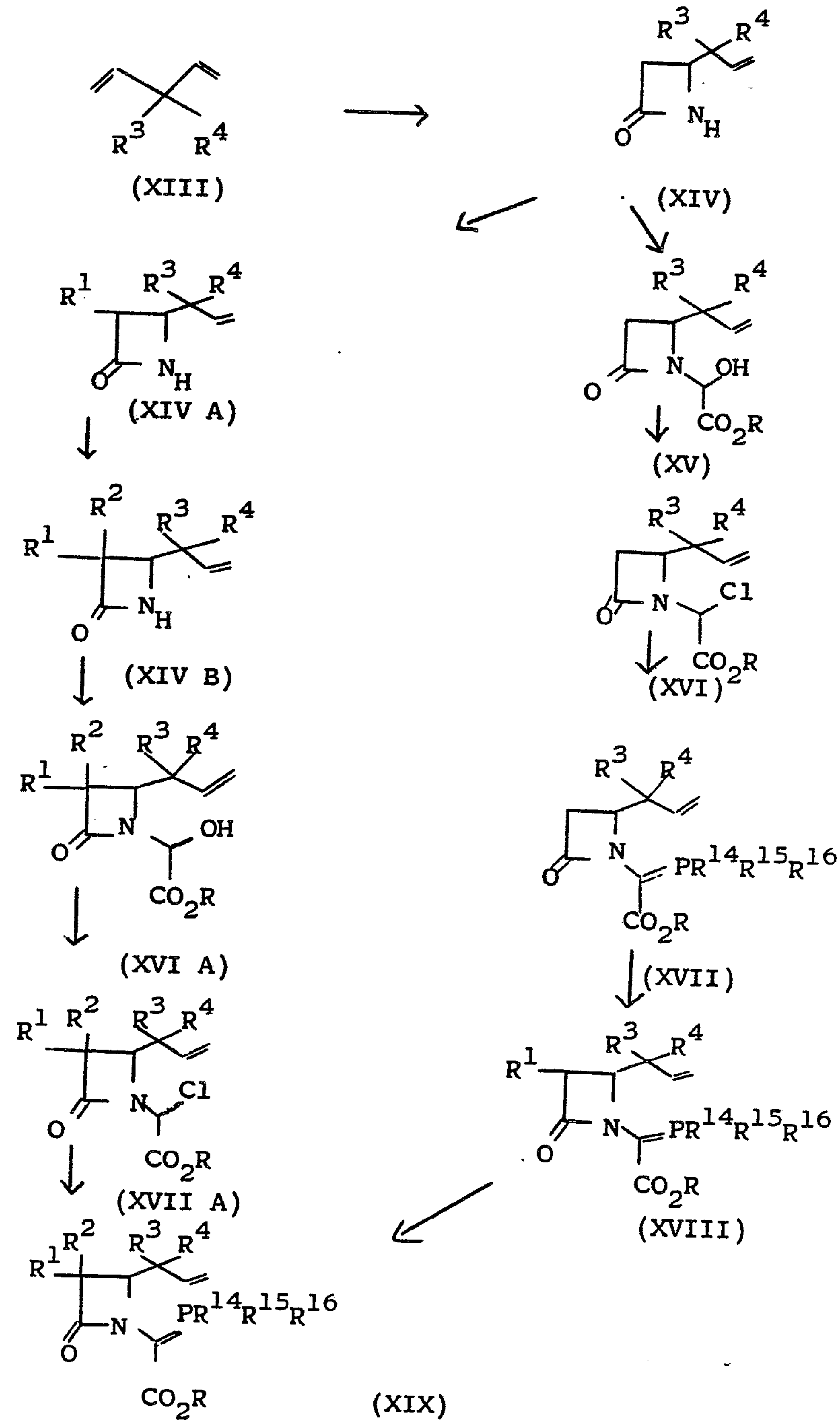

(XIII) (XIV) (XIV A) (XV) (XIV B) (XVI) (XVI A) (XVII) (XVII A) (XVIII) (XIX)

SCHEME 2

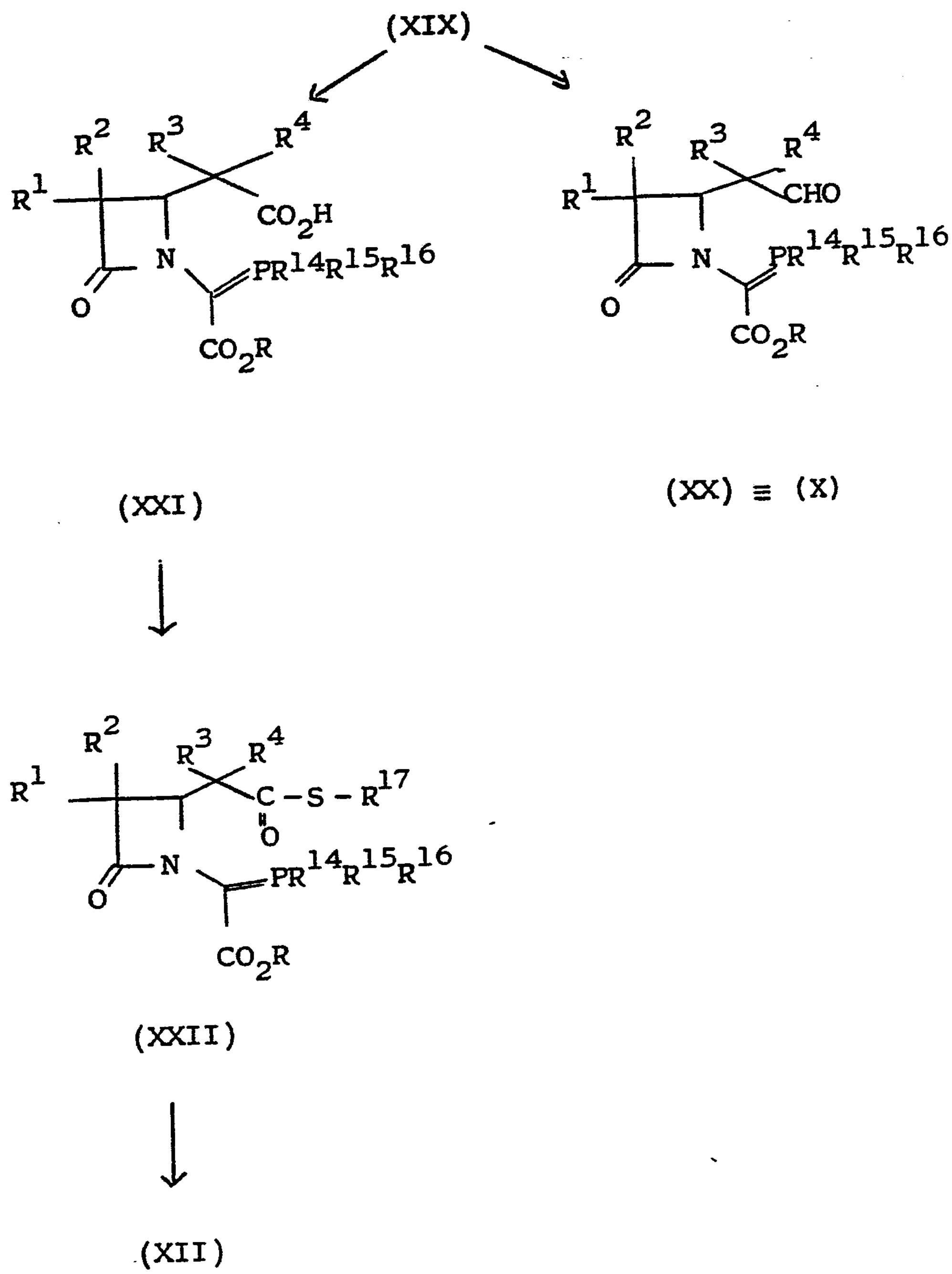

(XIX)

(XXI)

(XX) ≡ (X)

(XXII)

(XII)

The preparation of the compound (XXII) may be effected by the reaction of a compound of the formula (XXI):

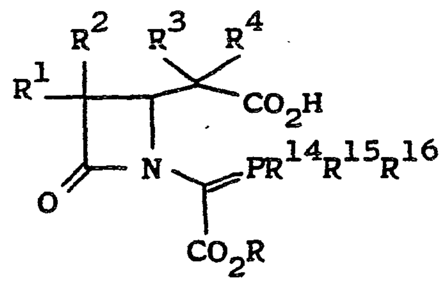

(XXI)

wherein $R^1$, $R^2$, $R^3$, $R^4$, $R^{14}$, $R^{15}$ and $R^{16}$ are as hereinbefore defined and R is an esterifying group, with a di-$(C_{1-6})$ alkylphosphorochloridate or thionylchloride and a tri-$(C_{1-6})$ alkylamine followed by reaction with a derivative of the formula (XXIII):

$$L^{\oplus} \ {}^{\ominus}SR^{17}$$ (XXIII)

wherein $L^{\oplus}$ is a lithium, sodium, silver or thallium (I) cation or an ammonium ion substituted by up to three $C_{1-6}$ alkyl groups, and $R^{17}$ is as defined hereinbefore.

When $L^{\oplus}$ is a substituted ammonium ion, it is preferably a tertiary ammonium ion, such as the triethylammonium ion. It is conveniently generated in situ by the reaction of a compound of the formula $HSR^{17}$ with an amine, preferably a tertiary amine.

Preferably $L^{\oplus}$ is a thallium I cation or a silver cation.

A particularly suitable diloweralkylphosphorochloridate is diethylphosphorochloridate.

A particularly suitable triloweralkylamine is triethylamine.

The reaction is generally carried out in an inert organic solvent such as tetrahydrofuran at a non-extreme temperature such as 0 to 40°C, for example 15-25°C.

The ester of the formula (XXI) may be prepared by the reaction of the compound of the formula (XIX).

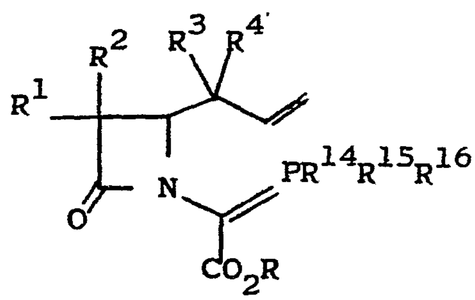

(XIX)

wherein $R^1$, $R^2$, $R^3$, $R^4$, $R^{14}$, $R^{15}$, $R^{16}$ and R are as hereinbefore defined, with ozone in the presence of trifluoroacetic acid followed by m-chloroperbenzoic acid.

The ozonolysis is generally performed at a depressed temperature such as -40 to -80°C, for example

about -70°C and in solution in an inert solvent such as methylene chloride. Excess ozone is removed by flushing with an inert gas and thereafter a solution of the peracid is added to the reaction mixture.

The preparation of the compound of the formula (XX) may be effected by the ozonolysis in the presence of excess trifluoroacetic acid of the ester of the formula (XIX):

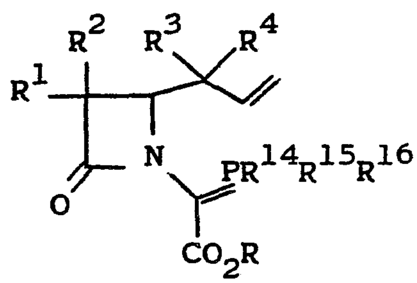

(XIX)

wherein $R^1$, $R^2$, $R^3$, $R^4$, $R^{14}$, $R^{15}$, $R^{16}$ and R are as hereinbefore defined, and thereafter neutralising the trifluoroacetic acid.

This ozonolysis is normally carried out in an inert solvent such as ethyl acetate.

The process is normally carried out a depressed temperature, for example -80° to -20°C, more usually at about -70° to -50°C. In general ozone is passed through the solution until a light blue colour is produced. At this point excess ozone may be removed by passing through an inert gas. The intermediate ozonide may now be reduced

by the addition of a conventional reducing agent such as triphenylphosphine. The trifluoroacetic acid should now be neutralised for example by the addition of a solution of a base as sodium bicarbonate solution at about 0°C.

Once the neutralization is complete the reaction is usually allowed to warm to ambient temperature under which conditions the compound spontaneously cyclises.

The compound of the formula (XIX) wherein $R^1$ and $R^2$ are not both hydrogen may be prepared by the mono- or di-alkylation or acylation of a compound of the formula (XVII):

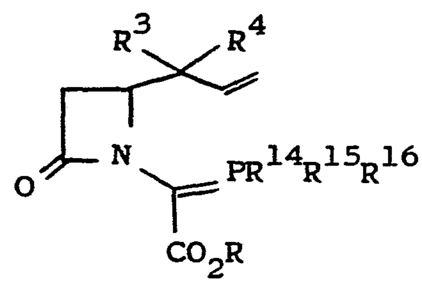

(XVII)

wherein $R^3$, $R^4$, $R^{14}$, $R^{15}$, $R^{16}$ and R are as hereinbefore defined. Suitably the compound of the formula (XVII) is treated with a strong base of low nucleophilicity such as butyl lithium, lithium di-iso-propylamide or lithium isopropyl-cyclohexylamide, in a solvent such as tetrahydrofuran, hexane, dimethoxyethane, dimethylformamide or hexamethyl-phosphorus triamide, normally at a temperature below 0°C, preferably -80°C to -60°C, using the desired electrophilic agent $R^1$-X wherein X is a leaving group such as mesylate, tosylate, chloro, bromo or iodo. Alternatively the electrophilic agent is an aldehyde or ketone. The process can of course be repeated with an a further alkylating or

acylating agent for example $R^2$-X in order to obtain the dialkylated or di-acylated species. A favoured acylating agent is acetylimidazole.

The compound of the formula (XVII) may be prepared by the reaction of $PR^{14}R^{15}R^{16}$ wherein $R^{14}R^{15}R^{16}$ are as hereinbefore defined and the compound of the formula (XVI):

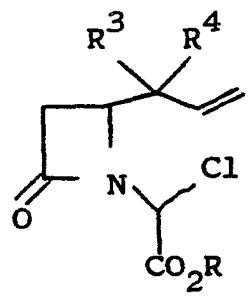

(XVI)

wherein $R^3$, $R^4$ and R are as hereinbefore defined.

This reaction is normally effected in the presence of at least one equivalent of a base of relatively low nucleophilicity such as 2,6-lutidine at an ambient temperature in a dry solvent such as dioxan, tetrahydrofuran or the like.

The ester of the compound of the formula (XVI) may be prepared from the corresponding ester of the carbinol of the formula (XV):

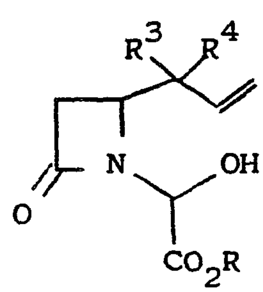

(XV)

wherein $R^3$, $R^4$ and R are as hereinbefore described, by reaction with thionyl chloride.

This reaction is also normally effected in the presence of at least one equivalent of a base of relatively low nucleophilicity in a dry solvent such as dioxan or tetrahydrofuran but in this instance the reaction is performed at a depressed temperature, for example -30 to -10°C.

The ester of the preceding carbinol may be prepared by the reaction of the compound of the formula (XIV):

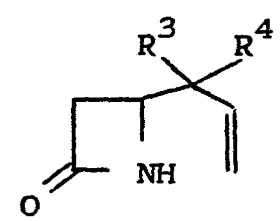

(XIV)

with a glyoxylic acid ester.

Normally this reaction is carried out in an inert solvent at an elevated temperature, for example in dry benzene under reflux.

The esters of the compounds of the formula (XV) may also be prepared by esterification of a salt of the compound of the formula (XV) in conventional manner.

Suitable methods include the reaction of alkali metal salt such as a sodium or potassium salt with a reactive halide or sulphonate ester such as a bromide, chloride, mesylate, tosylate or the like. Such esterifications may be carried out under conventional conditions, for example in dimethylformamide at room temperature.

The salt of compound of the formula (XV) may be prepared by neutralisation of the compound of the formula (XV) for example with an alkali metal carbonate or bicarbonate, for example sodium or potassium carbonate.

The compound of formula (XV) may be prepared by the reaction of glyoxylic acid with the compounds of the formula (XIV) as hereinbefore defined.

In an alternative the compound of the formula (XIV A) may be prepared by the reaction of the compound of the formula (XIV C) or (XIV D):

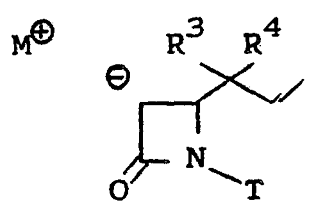

(XIV C)

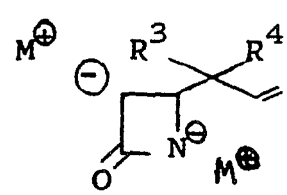

(XIV D)

wherein $R^3$ and $R^4$ are as hereinbefore defined, T is a protecting group for example a silyl protecting group such

as dimethyl-t-butylsilyl and $M^+$ is a counter-ion for example lithium; with an electrophilic reagent $R^1$-X. The compound of the formula (XIV A) may then be subjected to analogous treatment to afford the compound of the formula (XIV B). The compound (XIV B) is then taken through to compound (XIX) using techniques analogous to those hereinbefore described for (XIV)→(XVII).

Generally the compound of the formula (XIV D) is generated and utilised in situ. Then the compound of the formula (XIV) may be treated with two equivalents of n-butyl lithium in tetrahydrofuran at a low temperature. The dianion may be quenched by the addition of an alkylating or acylating agent.

Generally the compound of the formula (XIV C) is generated and used in situ. Thus the compound of the formula (XIV) may be treated with two equivalents of n-butyl lithium in tetrahydrofuran at a low temperature. The anion may be quenched by the addition of an alkylating or acylating agent. The protected group T may be joined and removed in conventional manner, for example 4-(1,1-dimethyl)allyl-1-t-butyldimethylsilylazetidin-2-one may be prepared by the reaction of 4-(1,1-dimethyl) allylazetidin-2-one with t-butyldimethylsilyl chloride and triethylamine in an inert solvent. The t-butyldimethylsilyl protecting group may be removed when necessary on treatment with potassium fluoride and a crown ether in an inert solvent.

The compounds of the formula (XXI) may also be prepared by the ozonolysis of an ester of a compound of the formula (XXX):

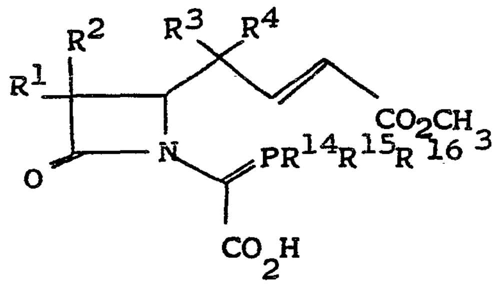

(XXX)

wherein $R^1$, $R^2$, $R^3$, $R^4$, $R^{14}$, $R^{15}$ and $R^{16}$ are as hereinbefore defined. The ozonolysis is generally performed in the presence of trifluoroacetic acid in dichloromethane or ethyl acetate at -70°C.

The ester of the compound of the formula (XXX) may be prepared from a compound of the (XXXI) in an analogous manner to that described for the process (XIV) ⟶ (XV) ⟶ (XVI) ⟶ (XVII):

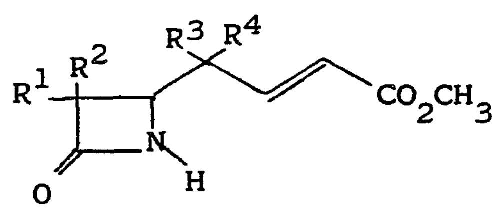

(XXXI)

wherein $R^1$ - $R^4$ are as hereinbefore defined.

The compound of the formula (XXXI) may be prepared by the oxidation of a compound (XXXII):

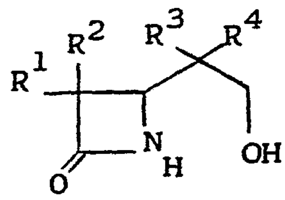

(XXXII)

wherein $R^1$ - $R^4$ are as hereinbefore defined, with pyridinium chlorochromate and thereafter reacting the thus produced aldehyde with $Ph_3P=CHCO_2CH_3$. The oxidation may be performed at ambient temperature in dichloromethane. When the oxidation is judged to be complete (for example by t.l.c) the reaction mixture may be filtered and the phosphorane added to the filtrate.

The compound of the formula (XXXII) may be prepared by mild acid hydrolysis of a compound of the formula (XXXIII):

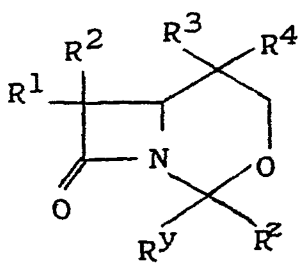

(XXXIII)

wherein $R^1$ - $R^4$ are as hereinbefore defined and $R^y$ and $R^z$ are independently $C_{1-6}$ alkyl such as methyl, or are joined together to form a substituted or unsubstituted spirocycloalkyl ring, for example spiro-cyclohexyl.

In one aspect compounds of the formula (XXXIII) wherein $R^1$ is hydrogen and $R^2$ is hydroxyethyl may be prepared by methods analogous to those described in UK Patent Application No. 2013667A. i.e. according to the Scheme:

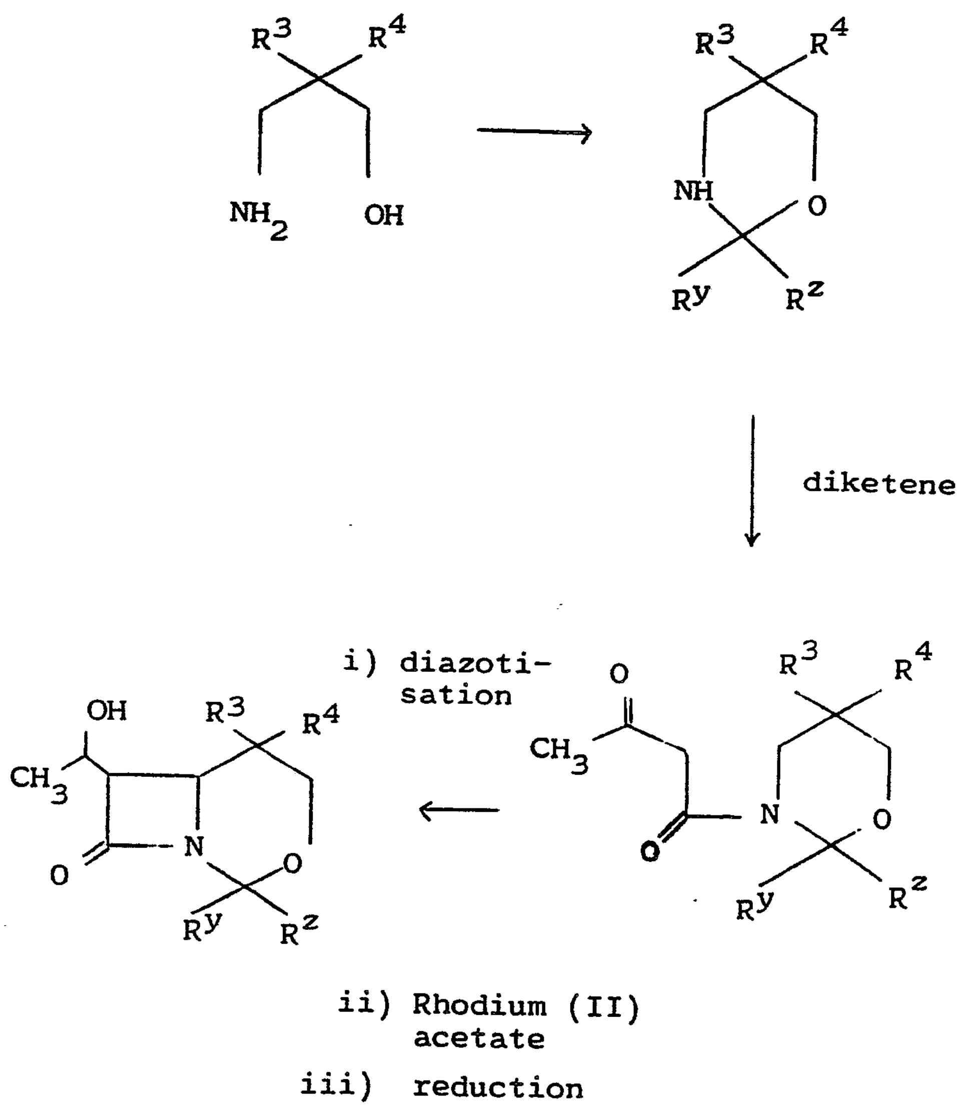

In a further aspect of this invention there is provided a process for preparation of a compound of the formula (XXIV):

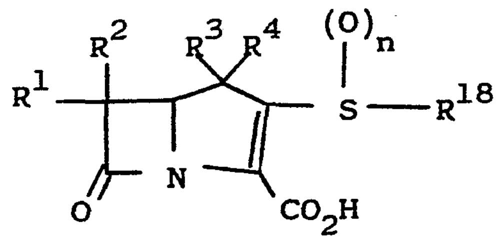

(XXIV)

or salt or ester thereof, wherein $R^1$, $R^2$, $R^3$, $R^4$ and n are as hereinbefore defined, and $R^{18}$ is $C_{1-6}$ alkyl, $C_{3-6}$ alkenyl wherein the double bond is not present on the carbon adjacent to the sulphur atom, $C_{3-6}$ alkynyl wherein the triple bond is not present on the carbon atom adjacent to the sulphur atom, aryl ($C_{1-6}$) alkyl, $C_{1-6}$ alkanoyl, aryl ($C_{1-6}$) alkanoyl, aryloxy ($C_{1-6}$) alkanoyl or arylcarbonyl, any of such groups $R^{18}$ being optionally substituted, which process comprises the reaction of an ester of a compound of the formula (XXV):

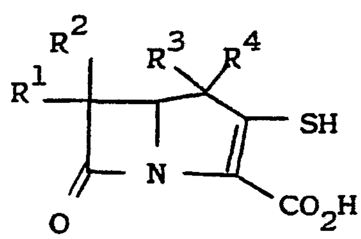

(XXV)

with a compound of the formula (XXVI):

$$X - R^{18} \qquad (XXVI)$$

wherein X is a leaving group, in the presence of an acid acceptor; and thereafter if desired.

i) converting the ester to an acid, salt or another ester,

ii) oxidising the sulphur atom to a SO group.

Suitable acid acceptors are carbonates and bicarbonates such as anhydrous potassium carbonate. The reaction is generally carried out in a dry polar solvent such as dimethylformamide. Suitably the reaction is performed at a non-extreme temperature, for example, -30°C to + 60°C, more suitable -10°C to + 40°C and preferably at ambient temperature.

Suitably X is a chlorine, bromine or iodine atom or is a sulphonate ester moiety such as tosylate or mesylate, of these values iodine and chlorine are preferred. In an alternative aspect when $R^{18}$ is a methyl or ethyl group, the leaving group X may be dimethyl ether or diethyl ether respectively. In other words X is derived from a trimethyloxonium salt or a triethyloxonium salt. Such salts are conveniently presented as their tetrafluoroborates. Such alkylations involving a trimethyloxonium or triethyloxonium salt are preferably performed in a halogenated hydrocarbon solvent for example dichloromethane or chloroform, at a depressed temperature for example -80°C to 0°C, more suitably -70°C to -30°C.

In another aspect of this invention there is provided a process for the preparation of a compound of the formula (XXVII):

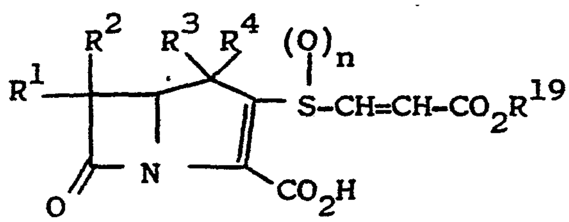

(XXVII)

or a salt or ester thereof wherein $R^1$, $R^2$, $R^3$, $R^4$ and n are hereinbefore defined, and $R^{19}$ is a hydrogen atom, salting-ion or esterifying group; which process comprises the reaction of an ester of a compound of the formula (XXV) with a compound of the formula (XXVIII):

$$H\text{-}C\equiv C\text{-}CO_2R^{19} \qquad (XXVIII)$$

in the presence of an acid acceptor; and thereafter if desired:

i) converting the ester to an acid, salt or another ester,

ii) oxidising the sulphur atom to a SO group.

Suitable acid acceptors are carbonates and bicarbonates such as anhydrous potassium carbonate. The reaction is generally carried out in a dry polar

solvent such as dimethylformamide. Suitably the reaction is performed at a non-extreme temperature, for example -30°C to + 60°C, more suitably -10°C to + 40°C and preferably at ambient temperature.

Compounds of the formula (XXV) or a salt or ester thereof may be prepared by the reaction of an ester of a compound of the formula (XXIX):

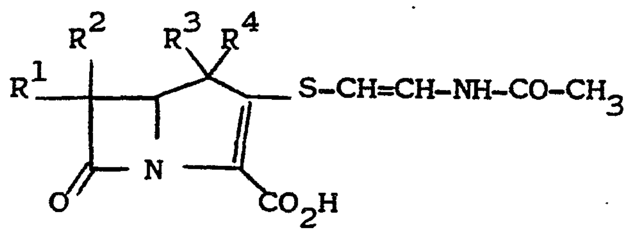

(XXIX)

wherein $R^1$, $R^2$, $R^3$ and $R^4$ are as hereinbefore defined; with a source of hypohalous acid, and thereafter if desired converting the ester to an acid, salt or another ester.

Suitably the reaction is performed at a non-extreme temperature such as - 15°C to + 25°C, preferably ambient. Solvents suitable in this reaction are inert organic solvents in the presence of moisture, for example moist acetone or dioxan. Suitably the hypohalous acid is hypobromous acid or hypochlorous acid, of these hypobromous acid is preferred. Suitably sources of hypohalous acids include N-bromoacetamide, N-chloroacetamide and N-bromopropionamide.

The following Examples illustrate the invention.

Example 1

4-(1,1-Dimethylallyl)azetidin-2-one

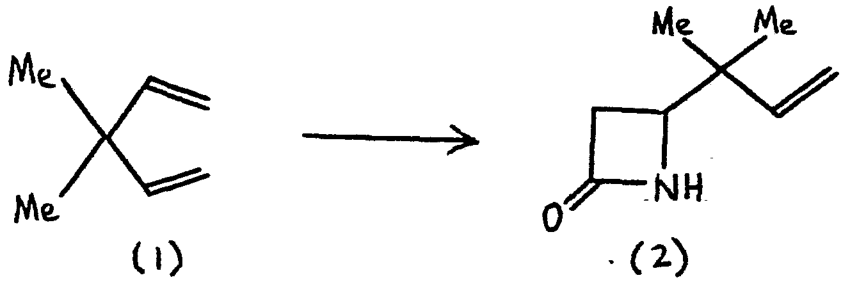

(1) (2)

A mixture of 3,3-dimethylpenta-1,4-diene(1)(0.68g) and chlorosulphonyl isocyanate (0.63ml) was kept in a sealed flask at room temperature for 2 days. The brown viscous product was dissolved in methylene chloride (6ml) and slowly added to a stirred mixture of water (10ml), sodium sulphite (1.20g), and methylene chloride (6ml), whilst maintaining the pH at 7 to 9 by addition of 10% aqueous potassium hydroxide. The organic layer was separated and the aqueous phase washed with ethyl acetate (10ml). The combined organic extracts were washed with brine, dried over sodium sulphate, and concentrated. Chromatography on silica gel 60 (<230 mesh ASTM) eluting with ethyl acetate/hexane 1:1 grading to ethyl acetate/ethanol 19:1 gave 4-(1,1-dimethylallyl)azetidin-2-one(2) as a colourless viscous liquid (0.15g); $\nu_{max.}$ ($CHCl_3$) 3380, 3220, 1750 and 1635$cm^{-1}$; $\delta$ ($CDCl_3$) 1.03 (6 H, s, $CH_3$'s), 2.66 (1 H, ddd, *J* 15, 3 and 1 Hz, 3-H), 2.85 (1 H, ddd, *J* 15, 5 and 2 Hz, 3-H), 3.50 ( 1 H, dd, *J* 5 and 3 Hz, 4-H), 5.0-5.2 (2 H, m, $C=CH_2$), 5.81 (1 H, dd, *J* 18 and 11 Hz, -CH=C), and 5.94 (1 H, br, NH).

Example 2

p-Nitrobenzyl [4-(1,1-dimethylallyl)-2-oxoazetidin-1-yl] triphenylphosphoranylideneacetate

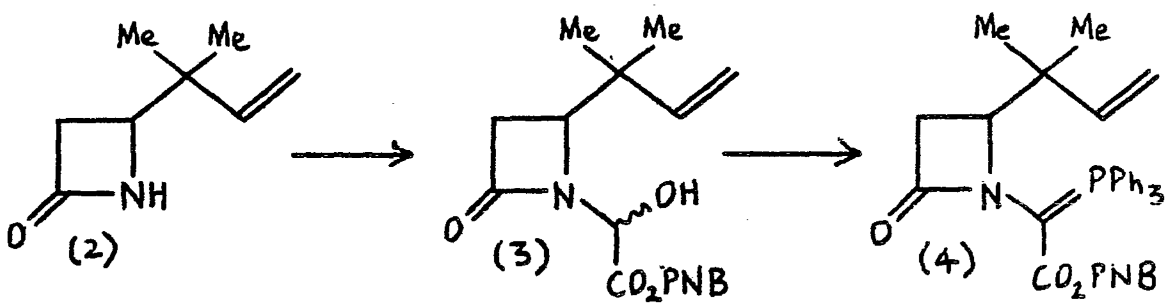

(2) (3) (4)

A suspension of p-nitrobenzyl glyoxylate (0.90g) in dry benzene (20 ml) was heated in a Dean and Stark apparatus for 0.5h. A benzene (5ml) solution of the azetidinone (2) (0.27g) was then added and the two components heated together at reflux for 4h. Concentration of the product followed by chromatography on silica gel 60 (< 230 mesh ASTM) eluting with ethylacetate/hexane 1:1 gave the mixed epimers of hydroxyester (3) as a waxy solid (0.63g); $\nu_{max.}(CHCl_3)$ 3500, 1740, 1605 and 1520cm$^{-1}$.

This compound (3) (0.62g) was dissolved in dry tetrahydrofuran (20ml) and stirred under argon in an ice-salt bath. It was treated with 2,6-lutidine (0.41ml) followed by thionyl chloride (0.26ml) and after 0.5h the reaction was filtered. The filtrate was concentrated, re-evaporated twice from toluene and then kept under high vacuum for 0.5h. The gum was dissolved in dry dioxane (20ml) and stirred under argon at room temperature. It was treated with triphenylphosphine (0.93g) and 2,6-lutidine (0.41ml) and the reaction mixture maintained at room temperature for 18h followed by 50°C for 20h. The product was concentrated and partitioned between ethyl acetate and brine. The organic

phase was separated, dried over sodium sulphate, concentrated, and chromatographed on silica gel 60 (< 230 mesh ASTM) eluting with ethylacetate/hexane 1:1 grading to 7:3. This gave the phosphorane (4) as a pale yellow foam (0.67g); $\nu_{max.}$ ($CHCl_3$) 1725, 1640, 1605 and 1520cm$^{-1}$.

Example 3

P-Nitrobenzyl 4,4-dimethyl-7-oxo-1-azabicyclo [3.2.0]hept-2-ene-2-carboxylate

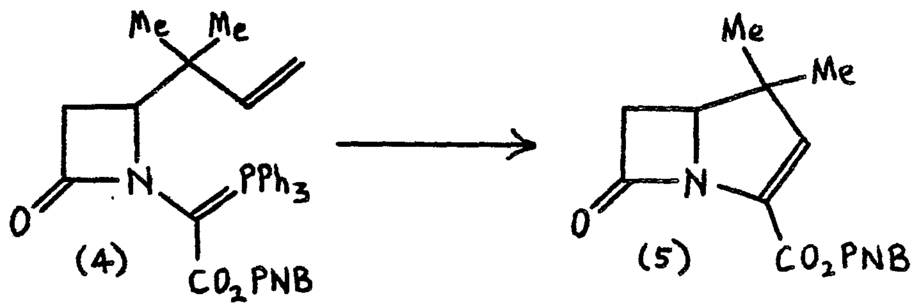

(4) (5)

A solution of the phosphorane (4) (0.67g) in ethyl acetate (40ml) was cooled to 0°C and trifluoracetic acid (3ml) was added. After a period of 10 min the solution was cooled to -70°C and ozone passed through it until it just became blue. The excess ozone was blown off in a stream of argon and a solution of triphenylphosphine (0.30g) in ethylacetate (2ml) was added. It was kept at -70°C for 0.5h and then allowed to warm to 0°C and neutralized by addition of saturated aqueous sodium bicarbonate (90ml). The organic layer was separated, washed with brine, dried over sodium sulphate, and then allowed to stand at room temperature for 4h. The solution was then concentrated and chromatographed on silica gel 60 (0.015-0.040mm) eluting with chloroform. This gave the bicyclic system (5) as a white solid (0.27g); m.p. 180-185°C (from chloroform/hexane); $\lambda_{max.}$ (EtOH) 267nm ($\varepsilon$14,800); $\nu_{max.}$ ($CHCl_3$) 1775, 1725, 1610 and 1520 $cm^{-1}$; $\delta$($CDCl_3$) 1.17 and 1.36 (6 H, 2s, $CH_3$'s), 3.07 (1 H, dd, *J* 17 and 3 Hz, 6-H), 3.33 (1 H, dd, *J* 17 and 6 Hz, 6-H), 3.90 (1 H, dd, *J* 6 and 3 Hz, 5-H), 5.28 and 5.43 (2 H, ABq, *J* 14 Hz, benzyl $CH_2$), 6.40 (1 H, s, 3-H), 7.62 and 8.24 (4 H, 2d, *J* 9 Hz, aromatic) (Found: C, 60.6; H, 5.3 ; N, 8.6% $C_{16}H_{16}N_2O_5$ requires C, 60.8; H, 5.1; N, 8.9%).

Example 4

p-Nitrobenzyl 4,4-dimethyl-3-ethylthio-7-oxo-1-azabicyclo[3.2.0.]heptane-2-carboxylate

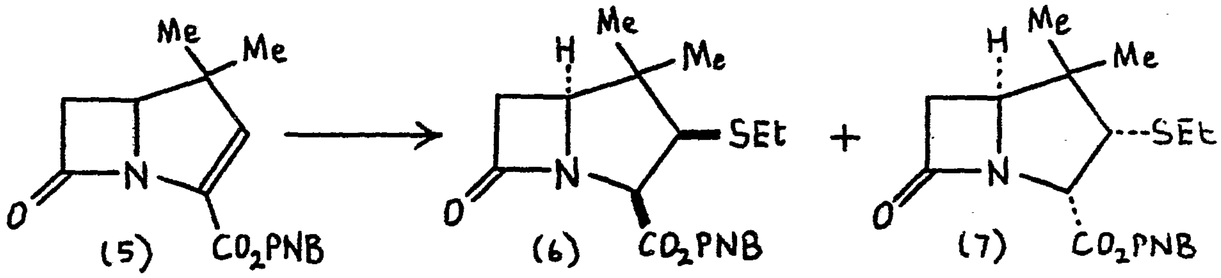

(5) (6) (7)

A suspension of the bicyclic compound (5) (0.23g) in dry N,N,-dimethylformamide (5ml) was stirred at room temperature and treated with ethanethiol (0.11ml) and powdered potassium carbonate (0.051g). After 0.5h, the excess thiol was blown off with argon and the solution was concentrated under high vacuum at < $30^{\circ}C$. The residue was partitioned between ethyl acetate and brine, and the organic phase was separated, dried over sodium sulphate and concentrated. Chromatography on a column of silica gel 60 (< 230 mesh ASTM) using ethyl acetate/hexane 3:7 as eluant gave the two isomers of the thiol addition product as colourless gums. The less polar product was the 2β,3β-isomer (6) (0.080g); $\nu_{max.}$ ($CHCl_3$) 1745, 1605 and 1520$cm^{-1}$; δ ($CDCl_3$) 0.88 and 1.21 (6 H, 2s, 4-$Me_2$), 1.17 (3 H, t, J 7 Hz, $CH_3$ of SEt), 2.48 and 2.49 (2 H, 2q, J 7Hz, inner part of $ABX_3$ for $SCH_2$), 2.74 (1 H, dd, J 16 and 2 Hz, 6-H), 3.11 (1 H, dd, J 16 and 5 Hz, 6-H), 3.32 (1 H, d, J 10 Hz, 3-H), 3.56 (1 H, dd, J 5 and 2 Hz, 5-H), 4.13 (1 H, d, J 10 Hz, 2-H), 5.33 (2 H, s, benzyl $CH_2$), and 7.58 and 8.25 (4 H, 2d, J 9 Hz, aromatic) (Found: $M^+$, 378.1216. $C_{18}H_{22}N_2O_5S$ requires M, 378.1249). The more polar product was the 2α, 3α-isomer (7) (0.140g); $\nu_{max.}$ ($CHCl_3$) 1750, 1605 and 1520$cm^{-1}$;

δ($CDCl_3$) 1.14 and 1.26 (6 H, 2s, 4-$Me_2$), 1.17 (3 H, t, _J_ 7 Hz, $CH_3$ of SEt), 2.53 and 2.54 (2 H, 2q, _J_ 7 Hz, inner part of $ABX_3$ for $SCH_2$), 2.81 (1 H, dd, _J_ 16 and 3 Hz, 6-H), 3.17 (1 H, dd, _J_ 16 and 5 Hz, 6-H), 3.23 (1 H, d, _J_ 7 Hz, 3-H), 3.75 (1 H, dd, _J_ 5 and 3 Hz, 5-H), 4.79 (1 H, d, _J_ 7 Hz, 2-H), 5.30 (2 H, s, benzyl $CH_2$) and 7.58 and 8.25 (4 H, 2d, _J_ 9 Hz, aromatic) (Found: _M_$^+$, 378.1249. $C_{18}H_{22}N_2O_5S$ requires _M_, 378.1249).

Example 5

p -Nitrobenzyl 4,4-dimethyl-3-ethylthio-7-oxo-1-azabicyclo[3.2.0]hept-2-ene-2-carboxylate

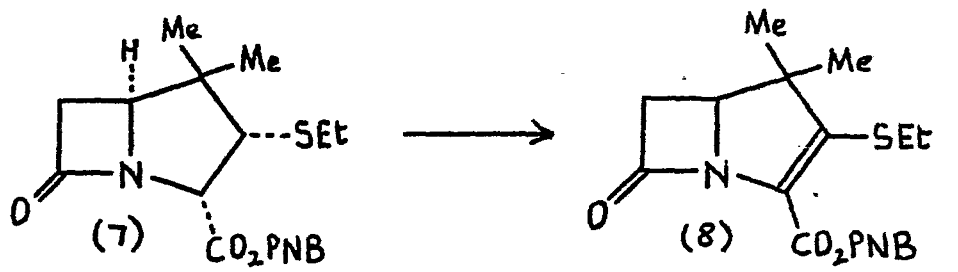

A solution of the 2α, 3α-isomer (7) (0.075g) in dry benzene (3ml) was stirred under argon and cooled to 5°C. This was treated with pyridine (0.017g) and iodobenzene dichloride (0.061g) and kept at 5°C for 1 h. A portion of 1,8-diazabicyclo[5.4.0]undec-7-ene (0.033g) was added and after a further 0.5h the reaction mixture was chromatographed on a column of silica gel 60 (0.015-0.040mm) eluting with ethylacetate/hexane 4:6. The unsaturated ester (8) was isolated as a colourless gum (0.027 g); $\lambda_{max.}$ (EtOH) 322nm (ε8600); $\nu_{max.}$ ($CHCl_3$) 1765, 1715, 1605, 1550 and 1525cm$^{-1}$; δ ($CDCl_3$) 1.19 and 1.32 (6 H, 2s, 4-$Me_2$), 1.20 (3 H, t, J 7 Hz, $CH_3$ of SEt), 3.02 (1 H, dd, J 16 and 3 Hz, 6-H), 3.04 (2 H, q, J 7 Hz, $SCH_2$), 3.36 (1 H, dd, J 16 and 6 Hz, 6-H), 3.81 (1 H, dd, J 6 and 3 Hz, 5-H), 5.29 and 5.47 (2 H, ABq, J 14 Hz, benzyl $CH_2$) and 7.67 and 8.24 (4 H, 2d, J 9 Hz, aromatic) (Found: $M^+$, 376.1069. $C_{18}H_{20}N_2O_5S$ requires M, 376.1090).

Example 6

Sodium 4,4-dimethyl-3-ethylthio-7-oxo-1-azabicyclo[3.2.0] hept-2-ene-2-carboxylate

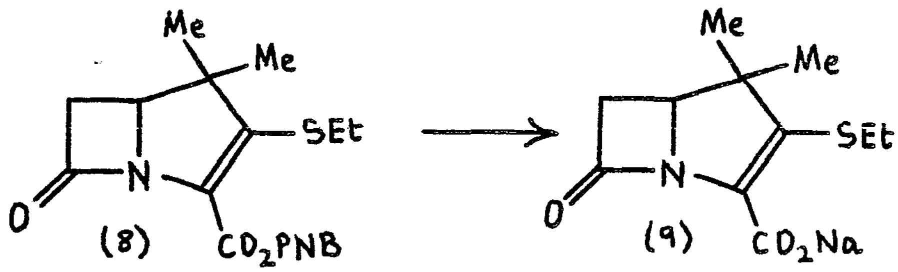

(8) (9)

A suspension of 5% palladium/charcoal catalyst (0.045g) in dioxane (5ml) and water (2.5ml) was prehydrogenated for 0.5h. A solution of the ester (8) (0.032g) in dioxane (2.5ml) was added and hydrogenation was carried out at atmospheric pressure for 2.5h. The product was treated with a solution of sodium bicarbonate (0.0071g) in water (0.5ml) and then filtered. The filtrate was concentrated until it just became turbid and was then washed twice with ethyl acetate. The aqueous solution of the sodium salt was purified by chromatography on Biogel P2 (200-400 mesh ASTM) eluting with water. This gave an aqueous solution containing the sodium salt (9) (0.0045g); $\lambda_{max.}$ ($H_2O$) 277nm.

Example 7

1-[3,3-Dimethyl-1-oxa-5-azaspiro[5.5]undecan-5-yl] butan-1,3-dione

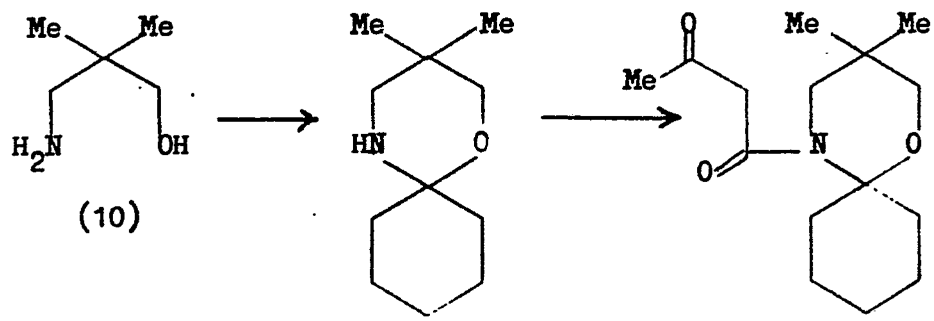

(10) (11) (12)

A solution of 3-amino-2,2-dimethylpropan-1-ol (10) (12.5 g) (P.F. Alewood, M. Benn and R. Reinfried, Can. J. Chem., 1974, 52, 4083) in dry benzene (120 ml) was treated with cyclohexanone (8.4 g) and heated at reflux in an apparatus with provision for water removal, for 6 h. The product was concentrated and than put under high vacuum for 0.5 h to give the crude 3,3-dimethyl-1-oxa-5-aza-spiro[5.5]undecane (11) as a yellow syrup. This material was redissolved in dry benzene (80 ml) and stirred and cooled in an ice bath. It was treated with diketene (7.2 ml) and maintained at 0°C for 1 h followed by room temperature for 2 h. The product was concentrated and chromatographed on silica gel 60 (<230 mesh ASTM) eluting with ethyl acetate/hexane 3:7 grading to 1:1 to give 1-[3,3-dimethyl-1-oxa-5-azaspiro[5,5]undecan-5-yl]butan-1,3-dione (12) (12.9 g); m.p. 55-58°C (hexane); $\nu_{max.}$ ($CHCl_3$) 3000, 2960, 2940, 2870, 1720, and 1635 $cm^{-1}$; $\delta$($CDCl_3$) 1.00 (6H, s, $C(CH_3)_2$) 2.23 (3H, s, $COCH_3$), 1.3 - 2.7 (10H, m, cyclohexyl), 3.05 (2H, s, $NCH_2$), 3.36 (2H, s, $OCH_2$), and 3.44 (2H, s, $COCH_2CO$) (Found: C, 67.6; H, 9.6; N, 5.1%. $C_{15}H_{25}NO_3$ requires C, 67.4; H, 9.4; N, 5.2%).

Example 8

1-[3,3-Dimethyl-1-oxa-5-azaspiro[5.5]undecan-5-yl]-2-diazobutan-1,3-dione

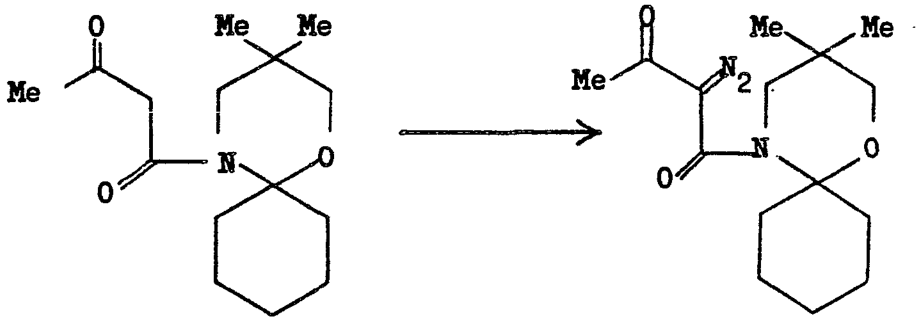

(12) (13)

A stirred solution of 1-[3,3-dimethyl-1-oxa-5-azaspiro[5.5]-undecan-5-yl]butan-1,3-dione (12) (13.3 g) in dry benzene (150 ml) was cooled to 0°C and treated with triethylamine (13.8 ml) followed by a solution of methanesulphonyl azide (12.0 g) in benzene (50 ml). The cooling bath was removed after 1 h and the solution stirred at room temperature for 4 days. The resulting solution was concentrated and chromatographed on silica gel 60 (<230 mesh ASTM) eluting with ethyl acetate/hexane 1:4 grading to 2:3 to give 1-[3,3-dimethyl-1-oxa-5-azaspiro[5,5]undecan-5-yl]-2-diazobutan-1,3-dione (13) (13.5 g), which could be recrystallized from hot hexane as pale yellow needles, m.p. 99-102°C (decomp.); $\nu_{max.}$ ($CHCl_3$) 3000, 2940, 2870, 2140, 2110, and 1645 $cm^{-1}$; $\delta$($CDCl_3$) 1.00 (6H, s, $C(CH_3)_2$), 1.4 - 2.5 (10H, m, cyclohexyl), 2.30 (3H, s, $CH_3CO$), 3.15 (2H, s, $NCH_2$), and 3.40 (2H, s, $OCH_2$).

Example 9

(6'RS,7'RS)-7'-Acetyl-5',5'-dimethylspiro[cyclohexane-1,2'-[3]oxa[1]azabicyclo[4.2.0]octane]-8'-one

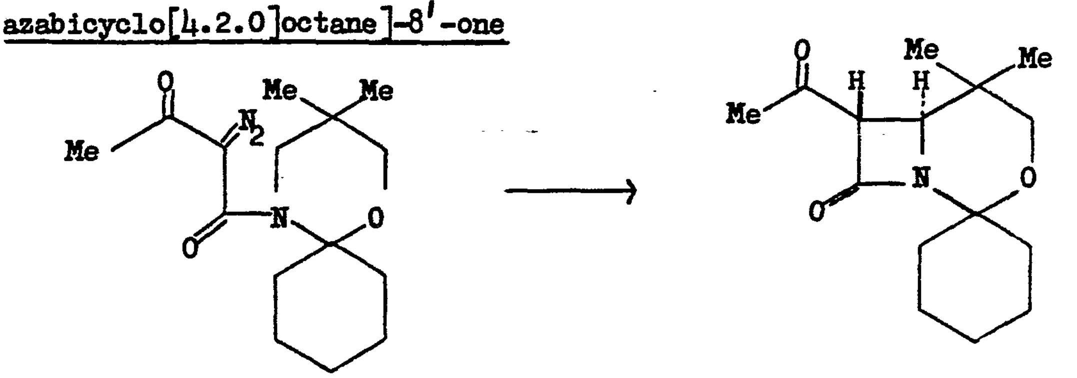

(13) (14)

Rhodium (II) acetate (1.00 g) was stirred in dry benzene (100 ml) at room temperature and a solution of 1-[3,3-dimethyl-1-oxa-5-aza-spiro[5.5]undecan-5-yl]-2-diazobutan-1,3-dione (13) (13.2 g) in benzene (50 ml) was added to it. The reaction mixture was stirred at room temperature for 2 days and the catalyst was then removed by filtration and the filtrate concentrated to small volume. It was chromatographed on silica gel 60 (<230 mesh ASTM) eluting with ethyl acetate/hexane 1:4 to give (6'RS,7'RS)-7'-acetyl-5',5'-dimethylspiro-[cyclohexane-1,2'-[3]oxa[1]azabicyclo[4.2.0]octane]-8'-one (14) (9.7 g); m.p. 98-99°C (hexane); $\nu_{max.}$ ($CHCl_3$) 3010, 2940, 2870, 1755, 1715, and 1560 $cm^{-1}$; $\delta$($CDCl_3$) 0.88 (3H, s, 5'-$CH_3$), 1.08 (3H, s, 5'-$CH_3$), 1.35-1.94 (10H, m, cyclohexyl), 2.33 (3H, s, $COCH_3$), 3.30 (1H, d, *J* 12Hz, 4'-H), 3.59 (1H, d, *J* 12Hz, 4'-H), 3.72 (1H, d, *J* 2Hz, 6'-H) and 3.93 (1H, d, *J* 2 Hz, 7'-H) (Found: C,67.8; H, 8.6; N, 5.2%; $M^+$ 265.1680. $C_{15}H_{23}NO_3$ requires C, 67.9; H, 8.7; N, 5.3%; *M* 265.1678).

Example 10

(6'RS, 7'RS)-5',5'-Dimethyl-7'-[(1SR)-1-hydroxyethyl]spiro[cyclohexane-1,2'-[3]oxa[1]azabicyclo[4.2.0]octane]-8'-one

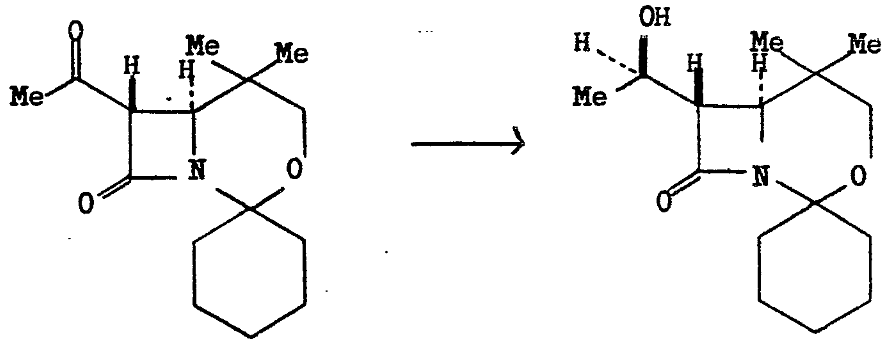

(14) (15)

A solution of (6'RS,7'RS)-7'-acetyl-5',5'-dimethylspiro[cyclohexane-1,2'-[3]oxa[1]azabicyclo[4.2.0]octane]-8'-one (14) (9.7g) in dry tetrahydrofuran (500 ml) was stirred at 0°C under argon and treated with a 0.5M solution in tetrahydrofuran of potassium tri-sec-butyl borohydride (88 ml). The cooling bath was removed and after 1 h the reaction mixture was quenched with water (30 ml). The tetrahydrofuran was distilled off under reduced pressure, and the residue dissolved in ethyl acetate, washed with brine, and dried over sodium sulphate. It was concentrated and chromatographed on silica gel 60 (<230 mesh ASTM) eluting with ethyl acetate/hexane 1:1 grading to 7:3, to give (6'RS,7'RS)-5',5'-dimethyl-7'-[(1SR)-1-hydroxyethyl]spiro[cyclohexane-1,2'-[3]oxa[1]azabicyclo[4.2.0]octane]-8'-one (15) (6.5 g), which could be recrystallized from ethyl acetate/hexane as small colourless needles; m.p. 108-110°C; $\nu_{max.}$ ($CHCl_3$) 3440, 3000, 2940, 2860, and 1735 $cm^{-1}$; $\delta$($CDCl_3$) 0.88 and 1.06 (6H, 2s, 5'-$(CH_3)_2$), 1.28 (3H, d, *J* 6Hz, $CH_3$-C-O), 1.3 - 2.4 (11H, m, cyclohexyl and OH), 2.95 (1H, dd, *J* 6 and 2Hz, 7'-H), 3.32 (1H, d, *J* 2Hz, 6'-H), 3.31 and 3.57 (2H, ABq, *J* 12Hz, 4'-$H_2$) and 4.1 - 4.2 (1H, br, CH-O) (Found: C, 67.7; H, 9.2; N, 5.2%. $C_{15}H_{25}NO_3$ requires C, 67.4; H, 9.4; N, 5.2%). The crude material

contained a small amount of the [(1RS)-1-hydroxyethyl]-diastereoisomers.

Example 11

(6′RS, 7′RS)-5′,5′-Dimethyl-7′-[(1SR)-1-(p-nitrobenzyloxycarbonyloxy)-ethyl]spiro[cyclohexane-1,2′-[3]oxa[1]azabicyclo[4.2.0]octane]-8′-one

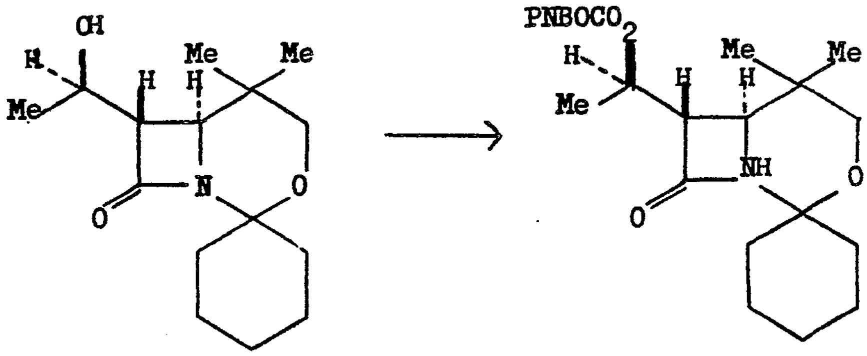

(15) (16)

A solution of (6′RS,7′RS)-5′,5′-dimethyl-7′-[(1SR-1-hydroxyethyl]spiro[cyclohexane-1,2′-[3]oxa[1]azabicyclo[4.2.0]octane]-8′-one (15) (6.49g) in dry tetrahydrofuran (300 ml) was stirred under argon at -70°C and treated with a 1.55 M hexane solution of n-butyl lithium (18.2 ml). After a period of 10 min, a solution of p-nitrobenzyl chloroformate (7.87 g) in tetrahydrofuran (90 ml) was added over 10 min, and the cooling bath removed. After a further 45 min the reaction mixture was treated with water (20 ml) and the solution concentrated. The residue was dissolved in ethyl acetate, washed with brine, dried over sodium sulphate, and concentrated. It was then chromatographed on silica gel 60 (<230 mesh), eluting with ethyl acetate/hexane 3:7 to give (6′RS, 7′RS)-5′,5′-dimethyl-7′-[(1SR)-1-(p-nitrobenzyloxycarbonyloxy)ethyl]spiro[cyclohexane-1,2′-[3]oxa[1]azabicyclo[4.2.0]octane]-8′-one (16) (7.52 g) as fine colourless needles, m.p. 85-88°C (ethyl acetate/hexane); $\nu_{max.}$ 3000, 2940 2860, 1745, 1615, and 1525cm$^{-1}$; $\delta$($CDCl_3$) 0.82 and 1.07 (6H, 2s, 5′-($CH_3$)$_2$), 1.45 (3H, d, J 6Hz, $CH_3$-C-O), 1.4 - 2.4 (10H, m, cyclohexyl), 3.08 (1H, dd, J 8 and 2Hz, 7′-H), 3.24 (1H, d, J 2Hz, 6′-H), 3.29 and 3.53 (2H, ABq, J 12Hz, 4′-$H_2$), 5.07 (1H, dq, J 8 and 6Hz, CH-O-), 5.25 (2H, s, benzylic $CH_2$), 7.54 and 8.24 (4H, 2d, J 9Hz, aromatic)

(Found: C, 61.8; H, 7.1; N 5.9%. $C_{23}H_{30}N_2O_7$ requires C, 61.9; H, 6.8; N, 6.3%.).

Example 12

(3RS,4RS)-4-(1,1-Dimethyl-2-hydroxyethyl)-3-[(1SR)-1-(p-nitrobenzyloxycarbonyloxy)ethyl]azetidin-2-one

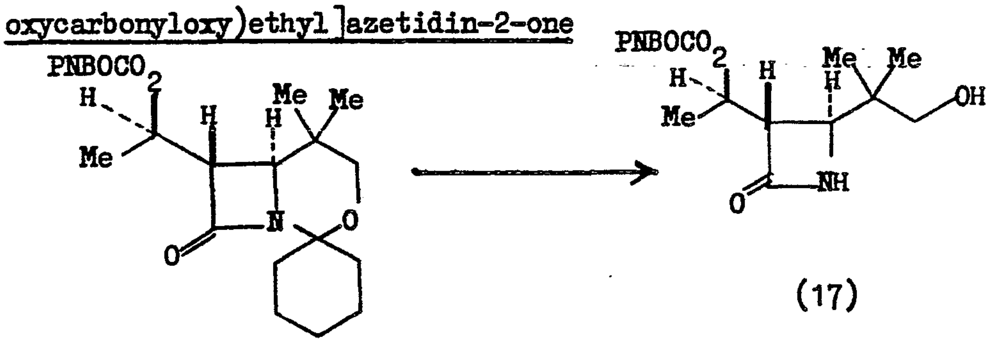

(17)

(16)

A solution of (6'RS,7'RS)-5',5'-dimethyl-7'-[(1SR)-1-(p-nitrobenzyloxycarbonyloxy)ethyl]spiro[cyclohexane-1,2'-[3]oxa[1]azabicyclo[4.2.0]octane]-8'-one (16) (7.42 g) in dioxane (250 ml) was treated with a solution of concentrated sulphuric acid (10 ml) in water (100 ml) This was heated at 80°C for 3 h and the resulting solution was cooled and neutralized with saturated aqueous sodium hydrogen carbonate. The solution was then concentrated until it just became turbid and was extracted with ethyl acetate (3 x 250 ml). The combined organic extracts were dried over sodium sulphate and concentrated to about 70 ml volume. Addition of an equal volume of hexane gave crystalline (3RS,4RS)-4-(1,1-dimethyl-2-hydroxyethyl)-3-[(1SR)-1-(p-nitrobenzyloxycarbonyloxy)ethyl]azetidin-2-one (17) (3.54 g); m.p. 176 - 179°C (from hot ethyl acetate); $\nu_{max.}$ (nujol) 3460, 3220, 1740, 1605, and 1520cm$^{-1}$; $\delta$[$(CD_3)_2SO$] 0.78 and 0.80 (6H, 2s, $C(CH_3)_2$), 1.31 (3H, d, J 6Hz, $CH_3$-C-O), 3.15 (2H, d, J 5Hz, $CH_2$-O), 3.18 (1H, dd, J 6 and 2Hz, 3-H), 3.37 (1H, d, J 2Hz, 4-H), 4.65 (1H, t, J 5Hz, OH), 4.93 (1H, quin, J 6Hz, >CH-O), 5.29 (2H, s, benzylic $CH_2$), 7.64 and 8.26 (4H, 2s, J 9Hz, aromatic), and 8.08 (1H, s, NH) (Found: C, 55.9; H, 6.1; N, 7.5%. $C_{17}H_{22}N_2O_7$ requires C, 55.7; H, 6.1; N, 7.7%).

Example 13

2-[(3RS,4RS)-3-[(1SR)-1-(p-Nitrobenzyloxycarbonyloxy)ethyl]-2-oxoazetidin-4-yl]-2-methylpropanal

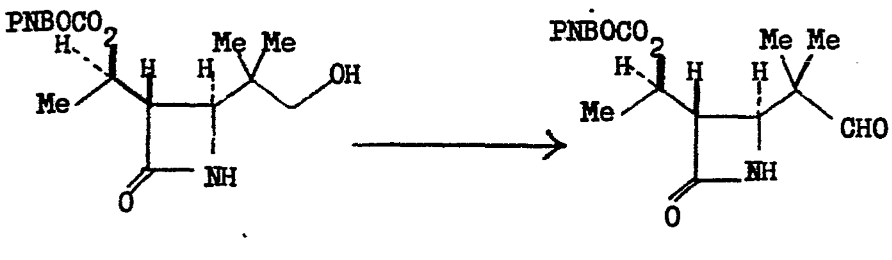

(17) (18)

A solution of (3RS,4RS)-4-(1,1-dimethyl-2-hydroxyethyl)-3-[(1SR)-1-(p-nitrobenzyloxycarbonyloxy)ethyl]azetidin-2-one (17) (0.200 g) in dry dimethylsulphoxide (1 ml) and toluene (1 ml) was treated with 1-ethyl-3-(3-dimethylaminopropyl)carbodiimide (0.314 g), pyridine (0.151 g) in toluene (0.5 ml), and trifluoroacetic acid (0.031 g) in toluene (0.5 ml). It was stirred under argon at room temperature for 4 h, and water and ethyl acetate were then added. The organic phase was separated and the aqueous phase washed with further ethyl acetate. The combined organic solutions were washed with 2N hydrochloric acid, aqueous sodium hydrogen carbonate, brine, and then dried over sodium sulphate. The solution was concentrated and chromatographed on silica gel 60 (<230 mesh ASTM) eluting with ethyl acetate/hexane 6:4 to give 2-[(3RS,4RS)-3-[(1SR)-1-(p-nitrobenzyloxycarbonyloxy)ethyl]-2-oxoazetidin-4-yl]-2-methylpropanal (18) (0.175 g); m.p. 135-137°C (ethyl acetate/hexane); $\nu_{max.}$ ($CHCl_3$) 3420, 3030, 2970, 2870, 2810, 2710, 1760, 1730 sh, 1610 and 1525 $cm^{-1}$; δ ($CDCl_3$) 1.14 and 1.16 (6H, 2s, $C(CH_3)_2$), 1.48 (3H, d, J 7Hz, $CH_3$-C-O), 3.12 (1H, dd, J 8 and 2 Hz, 3-H), 3.75 (1H, d, J 2Hz, 4-H), 5.12 (1H, dq, J 8 and 7 Hz, >CH-O), 5.27 (2H, s, benzylic $CH_2$), 5.95 (1H, br s, NH), 7.55 and 8.25 (4H, 2d, J 9Hz, aromatic) and 9.48 (1H, s, CHO) (Found: C, 55.8; H, 5.5; N, 7.6%. $C_{17}H_{20}N_2O_7$ requires C, 56.0; H, 5.5; N, 7.7%).

Example 14

Methyl (E)-4-[(3RS,4RS)-3-[(1SR)-1-(p-nitrobenzyloxycarbonyloxy)-ethyl]-2-oxoazetidin-4-yl]-4-methylpent-2-enoate

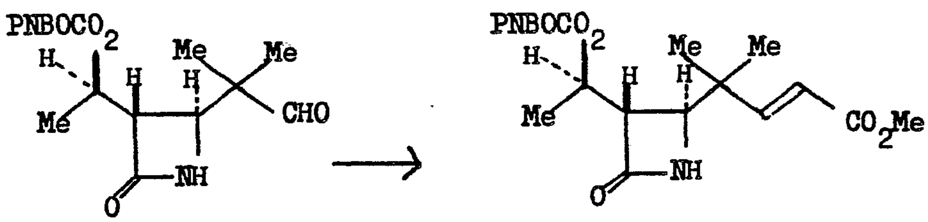

(18) (19)

A stirred mixture of 2-[(3RS,4RS)-3-[(1SR)-1-(p-nitrobenzyloxy-carbonyloxy)ethyl]-2-oxoazetidin-1-yl]-2-methylpropanal (18) (0.150 g) and methoxycarbonylmethylenetriphenylphosphorane (0.206 g) in dry benzene (10 ml) was heated under reflux for 12 h. The resulting solution was concentrated to small bulk and chromatographed on silica gel 60 (<230 mesh ASTM) eluting with ethyl acetate/hexane 1:1 to produce methyl (E)-4-[(3RS,4RS)-3-[(1SR)-1-(p-nitrobenzyloxy carbonyloxy)ethyl]-2-oxoazetidin-4-yl]-4-methylpent-2-enoate (19) (0.168 g); m.p. 118-120°C (benzene/hexane); $\nu_{max.}$ ($CHCl_3$) 3400, 2960, 1760, 1655, 1610 and 1520 $cm^{-1}$; $\delta$ ($CDCl_3$) 1.11 (6H, s, $C(CH_3)_2$), 1.45 (3H, d, J 7Hz, $CH_3$-C-O), 3.03 (1H, dd, J 7 and 2 Hz, 3-H), 3.53 (1H, d, J 2 Hz, 4-H), 3.74 (3H, s, $OCH_3$), 5.11 (1H, quin, J 7Hz, >CH-O), 5.26 (2H, broadened s, benzylic $CH_2$), 5.93 (1H, br.s, NH), 5.85 and 6.91 (2H, 2d, J 16 Hz, CH=CH), 7.55 and 8.25 (4H, 2d, J 9Hz, aromatic) (Found: C, 57.5; H, 5.6; N, 6.6%. $C_{20}H_{24}N_2O_8$ requires C, 57.1; H, 5.8; N, 6.7%).

Example 15

p-Nitrobenzyl 2-[(3RS, 4RS)-4-[(E)-1,1-dimethyl-3-methoxycarbonylprop-2-enyl]-3-[(1SR)-1-(p-nitrobenzyloxycarbonyloxy)ethyl]-2-oxo-azetidin-1-yl]-2-hydroxyacetate

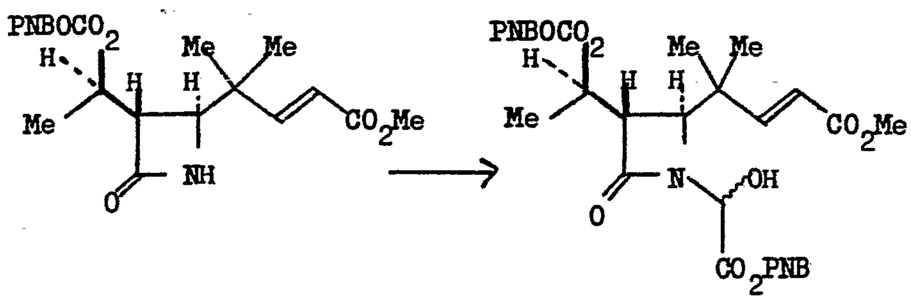

(19) (20)

A suspension of p-nitrobenzyl glyoxylate monohydrate (2.00 g) in benzene (120 ml) was heated in a Dean and Stark apparatus for 0.5 h to obtain a clear solution. To this was added methyl (E)-4-[(3RS, 4RS)-3-[(1SR)-1-(p-nitrobenzyloxycarbonyloxy)ethyl]-2-oxo-azetidin-4-yl]-4-methylpent-2-enoate (19) (2.47 g), and the solution was heated under reflux for 24 h. A small amount of water was added to precipitate excess glyoxylate, and the mother liquors were concentrated to small bulk. This was chromatographed on silica gel 60 (<230 mesh ASTM) eluting with chloroform to yield p-nitrobenzyl 2-[(3RS, 4RS)-4-[(E)-1,1-dimethyl-3-methoxycarbonylprop-2-enyl]-3-[(1SR)-1-(p-nitrobenzyloxycarbonyloxy)ethyl]-2-oxoazetidin-1-yl]-2-hydroxyacetate (20) (2.99 g) as a solid mixture of two diastereoisomers, which could be crystallized from benzene/hexane; m.p. 120 - 126°C; $\nu_{max.}$ ($CHCl_3$) 3520, 3050, 2970, 1760, 1655, 1610 and 1525cm$^{-1}$; δ ($CDCl_3$) 1.11 (6H, s, $C(CH_3)_2$), 1.34 (3H, d, J 7Hz, $CH_3$-C-O), 2.98 (1H, dd, J 8 and 2 Hz, 3-H), 3.65 (1H, d, J 2Hz, 4-H), 3.69 (3H, s, $OCH_3$), 4.8 - 5.0 (1H, m, >CH-O), 4.98 (1H, s, N-CH-O), 5.20 and 5.30 (4H, 2s, benzylic $CH_2$'s), 5.83 and 6.94 (2H, 2d, J 16 Hz, CH=CH), 7.53 and 8.22 (8H, 2d, J 9Hz, aromatics) (Found: C,55.4; H, 5.0; N, 6.3%. $C_{29}H_{31}N_3O_{13}$ requires C, 55.[illegible]; H, 5.0; N, 6.7%).

Example 16

p-Nitrobenzyl 2-[(3RS, 4RS)-4-[(E)-1,1-dimethyl-3-methoxycarbonylprop-2-enyl]-3-[(1SR)-1-(p-nitrobenzyloxycarbonyloxy)ethyl]-2-oxoazetidin-1-yl]-2-(triphenylphosphoranylidene)acetate

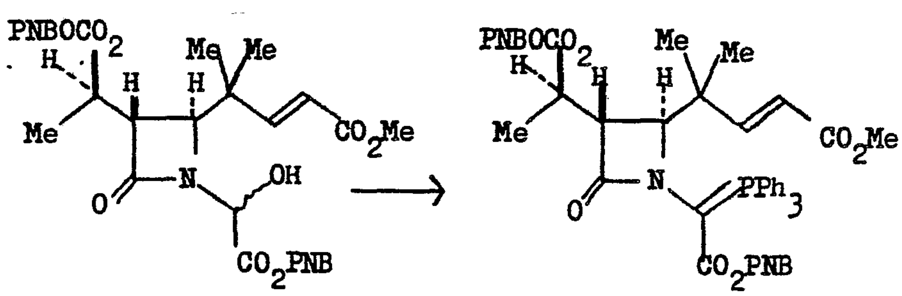

(20) (21)

The diastereoisomeric mixture of p-nitrobenzyl 2-[(3RS, 4RS)-4-[(E)-1,1-dimethyl-3-methoxycarbonylprop-2-enyl]-3-[(1SR)-1-(p-nitrobenzyloxycarbonyloxy)ethyl]-2-oxoazetidin-1-yl]-2-hydroxyacetates (20) (2.53 g) was dissolved in dry tetrahydrofuran (50 ml) and stirred at -20°C under argon. It was treated with 2,6-lutidine (0.94 ml) followed by thionyl chloride (0.59 ml) and after a period of 0.5 h the product was filtered and the filtrate concentrated. The gum was re-evaporated twice from toluene and then put under high vacuum for 0.5 h. The resulting pale yellow gum was dissolved in dry dioxane (30 ml) and triphenylphosphine (2.11 g) and 2.6-lutidine (0.94 ml) were added. The reaction mixture was stirred under argon for 3 days at 70°C. The product was diluted with ethyl acetate, washed with water, dried over sodium sulphate and concentrated. This was than chromatographed on silica gel 60 (<230 mesh ASTM) eluting with ethylacetate/hexane 1:1 to give p-nitrobenzyl 2-[(3RS, 4RS)-4-[(E)-1,1-dimethyl-3-methoxycarbonylprop-2-enyl]-3-[(1SR)-1-(p-nitrobenzyloxycarbonyloxy)-ethyl]-2-oxoazetidin-1-yl]-2-(triphenylphosphoranylidene)acetate (21) as a crisp yellow foam (2.16 g); $\nu_{max.}$ ($CHCl_3$) 2990, 1745, 1655, 1610 and 1525 $cm^{-1}$.

Example 17

p-Nitrobenzyl 2-[(3RS,4RS)-4-(1-carboxy-1-methylethyl)-3-[(1SR)-1-(p-nitrobenzyloxycarbonyloxy)ethyl]-2-oxoazetidin-1-yl]-2-(triphenylphosphoranylidene)acetate

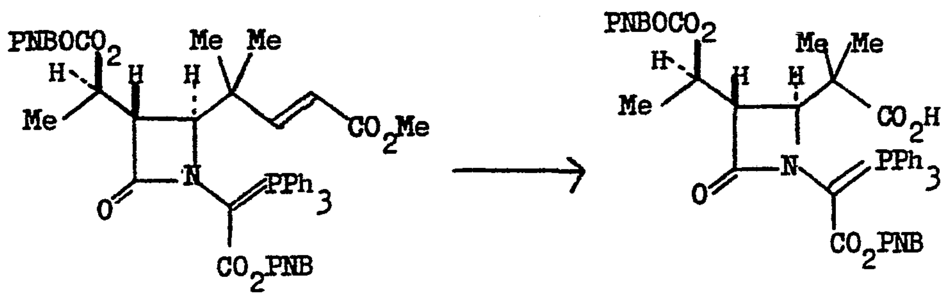

(21) (22)

A solution of p-nitrobenzyl 2-[(3RS,4RS)-4-[(E)-1,1-dimethyl-3-methoxycarbonylprop-2-enyl]-3-[(1SR)-1-(p-nitrobenzyloxycarbonyloxy)-ethyl]-2-oxoazetidin-1-yl]-2-(triphenylphosphoranylidene)acetate (21) (0.34 g) in dry dichloromethane (15 ml) was cooled to 0°C and trifluoroacetic acid (1.5 ml) was added. After 15 min it was cooled to -50°C and ozone was bubbled through the solution until it just became blue. The excess ozone was blown out with argon, and a solution of m-chloroperbenzoic acid (0.075 g) in dichloromethane (1 ml) was added. The reaction mixture was stirred overnight at room temperature and then evaporated and re-evaporated from toluene. Chromatography of the residue on silica gel 60 (<230 mesh) using ethyl acetate/hexane 7:3 grading to 1:0 gave p-nitrobenzyl 2-[(3RS,4RS)-4-(1-carboxy-1-methylethyl)-3-[(1SR)-1-(p-nitrobenzyloxycarbonyloxy)ethyl]-2-oxoazetidin-1-yl]-2-(triphenylphosphoranylidene)-acetate (22) (0.13 g) as a colourless gum which could be crystallized from ethyl acetate as the monosolvate; m.p. 148 - 153°C; $\nu_{max.}$ ($CHCl_3$) 2980 br, 1745, 1605 and 1525cm$^{-1}$ (Found: C, 62.5; H, 5.2; N, 4.6%. $C_{44}H_{40}N_3O_{12}P$, $C_4H_8O_2$ requires C, 62.5; H, 5.3; N, 4.6%).

Example 18

p-Nitrobenzyl 2-[(3RS,4RS)-3-[(1SR)-1-(p-nitrobenzyloxycarbonyloxy)-ethyl]-2-oxo-4-[1-(pyrimidin-2-ylthiocarbonyl)-1-methylethyl]-azetidin-1-yl]-2-(triphenylphosphoranylidene)acetate

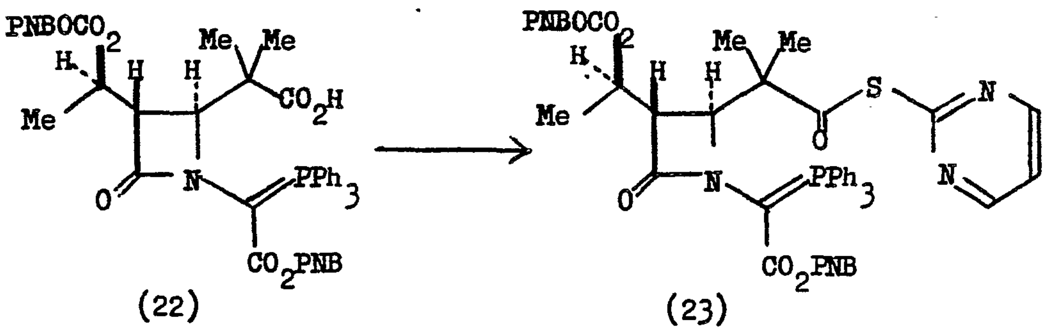

(22) (23)

A stirred suspension of pyrimidine-2-thiol (0.117 g) in dry tetrahydrofuran (6 ml) at room temperature was treated with a 1.6 M hexane solution of n-butyl lithium (0.65 ml). The thiol dissolved and then the lithium thiolate precipitated to give a yellow suspension.

p-Nitrobenzyl 2-[(3RS,4RS)-4-(1-carboxy-1-methylethyl)-3-[(1SR)-1-(p-nitrobenzyloxycarbonyloxy)ethyl]-2-oxoazetidin-1-yl]-2-(triphenylphosphoranylidene)acetate (22) (0.733 g) was stirred in dry tetrahydrofuran (20 ml) at room temperature under argon, and triethylamine (0.105 g) and diethylchlorophosphate (0.181 g) were added. This was stirred for 6 h and then the lithium pyrimidine-2-thiolate suspension was transferred to it. The reaction mixture was stirred overnight and then concentrated and the residue partitioned between ethyl acetate and brine. The organic phase was washed with brine, dried over sodium sulphate, concentrated, and chromatographed on silica gel 60 (<230 mesh ASTM) eluting with ethyl acetate/hexane 7:3 grading to 1:0. This gave p-nitrobenzyl 2-[(3RS,4RS)-3-[(1SR)-1-(p-nitrobenzyloxycarbonyloxy)ethyl]-2-oxo-4-[1-(pyrimidin-2-ylthiocarbonyl)-1-methylethyl]azetidin-1-yl]-2-(triphenylphosphoranylidene)acetate (23) (0.460 g) as a colourless gum; $\nu_{max.}$ ($CHCl_3$) 2990, 1745, 1710 sh, 1605, 1550 and 1520 $cm^{-1}$.

Example 19

p-Nitrobenzyl (5RS,6RS)-4,4-dimethyl-6-[(1SR)-1-(p-nitrobenzyloxy-carbonyloxy)ethyl]-3-(pyrimidin-2-ylthio)-7-oxo-1-azabicyclo[3.2.0]-hept-2-en-2-carboxylate

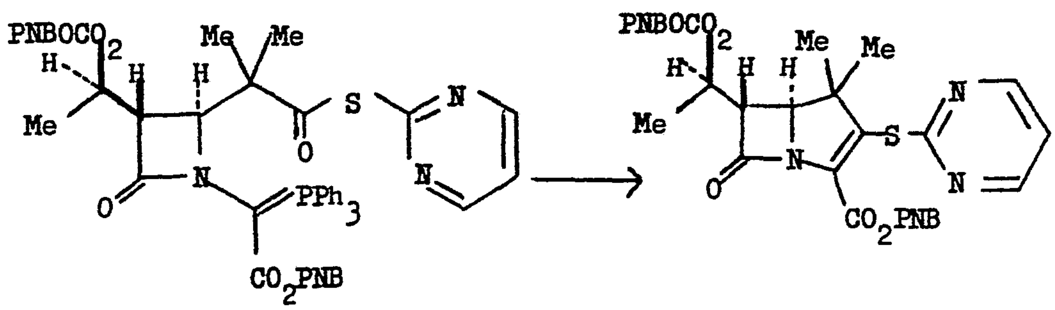

(23) (24)

p-Nitrobenzyl 2-[(3RS,4RS)-3-[(1SR)-1-(p-nitrobenzyloxycarbonyloxy)ethyl]-2-oxo-4-[(1-(pyrimidin-2-ylthiocarbonyl)-1-methylethyl]azetidin-1-yl]-2-(triphenylphosphoranylidene)acetate (23) (0.490 g) was dissolved in dry toluene (500 ml) and heated under reflux for 36 h in a Dean and Stark apparatus. The resulting solution was concentrated to small bulk and chromatographed on silica gel 60 (<230 mesh ASTM) using ethyl acetate/hexane 1:1 as eluant. This gave p-nitrobenzyl (5RS, 6RS)-4,4-dimethyl-6-[(1SR)-1-(p-nitrobenzyloxycarbonyloxy)ethyl]-3-(pyrimidin-2-ylthio)-7-oxo-1-azabicyclo[3.2.0]hept-2-en-2-carboxylate (24) (0.236 g) as a colourless gum; $\lambda_{max.}$ (EtOH) 253 and 322 sh nm; $\nu_{max.}$ ($CHCl_3$) 3030, 2990, 2960, 2860, 1780, 1740, 1605, 1560, 1550 and 1525 $cm^{-1}$; $\delta$ ($CDCl_3$) 1.19 and 1.30 (6H, 2s, 4-$(CH_3)_2$), 1.51 (3H, d, *J* 7Hz, $CH_3$-C-O), 3.56 (1H, dd, *J* 7 and 3 Hz, 6-H), 3.99 (1H, d, *J* 3Hz, 5-H), 5.24 (1H, quin, *J* 7Hz, -CH-O), 5.27 (2H, s, benzylic $CH_2$), 5.15 and 5.30 (2H, ABq, *J* 12 Hz, benzylic $CH_2$), 6.95 (1H, t, *J* 5 Hz, pyrimidinyl 5-H), 7.49, 7.56, 8.15 and 8.24 (8H, 4d, *J* 9Hz, nitrophenyls) and 8.39 (2H, d, *J* 5Hz, pyrimidinyl 4-H and 6-H) (Found: *M*$^+$, 649.148 9. $C_{30}H_{27}N_5O_{10}S$ requires *M*, 649.147 6).

Example 20

Sodium (5RS,6RS)-4,4-dimethyl-6-[(1SR)-1-hydroxyethyl]-3-(pyrimidin-2-ylthio)-7-oxo-1-azabicyclo[3.2.0]hept-2-en-2-carboxylate

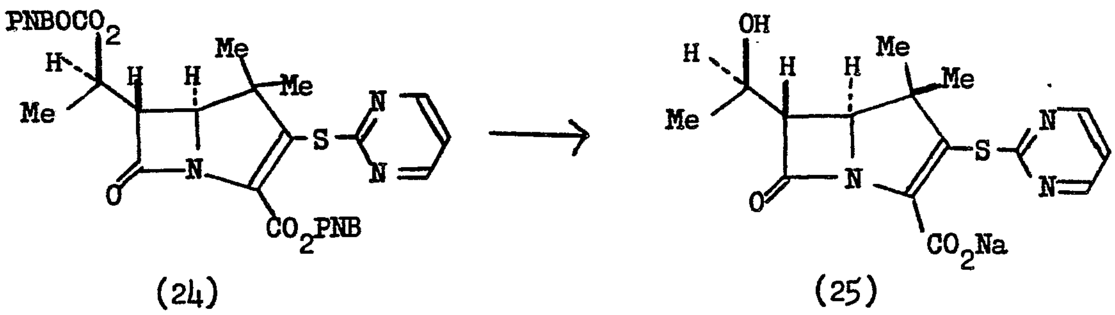

(24) (25)

A suspension of 10% palladium-charcoal catalyst (0.036 g) in dioxane (10 ml) and water (2.5 ml) was hydrogenated for 0.5 h at atmospheric pressure. To this was added a solution of p-nitrobenzyl (5RS,6RS)-4,4-dimethyl-6-[(1SR)-1-(p-nitrobenzyloxycarbonyloxy)ethyl]-3-(pyrimidin-2-ylthio)-7-oxo-1-azabicyclo[3.2.0]hept-2-en-2-carboxylate (24) (0.036 g) in dioxane (2.5 ml), and also 0.05 M phosphate buffer (1 ml). The mixture was hydrogenated at atmospheric pressure until deprotection was complete (about 3 h) and the product was treated with a solution of sodium hydrogen carbonate (0.0046 g) in water (1 ml). This was then filtered and the filtrate concentrated until it just became turbid. It was washed with ethyl acetate (2 x 5 ml) and the aqueous solution then passed through a column of Biogel P2 eluting with water. The fractions containing product were partially concentrated under reduced pressure and then freeze dried to give sodium (5RS,6RS)-4,4-dimethyl-6-[(1SR)-1-hydroxyethyl]-3-(pyrimidin-2-ylthio)-7-oxo-1-azabicyclo[3.2.0]hept-2-en-2-carboxylate (25) (0.018 g); $\lambda_{max.}$ ($H_2O$) 248 and 278 nm; $\delta$ ($D_2O$) 1.13 and 1.20 (6H, 2s, 4-$(CH_3)_2$), 1.28 (3H, d, J 6Hz, $CH_3$-C-O), 3.52 (1H, dd, J 6 and 3 Hz, 6-H), 3.99 (1H, d, J 3Hz, 5-H), 4.28 (1H, quin, J 6Hz, -CH-O), 7.25 (1H, t, J 5Hz, pyrimidinyl 5-H) and 8.54 (2H, d, J 5Hz, pyrimidinyl 4-H and 6-H).

Example 21

p-Nitrobenzyl (5RS,6RS)-4,4-dimethyl-6-[(1SR)-1-(p-nitrobenzyloxycarbonyloxy)ethyl]-7-oxo-1-azabicyclo[3.2.0]hept-2-en-2-carboxylate

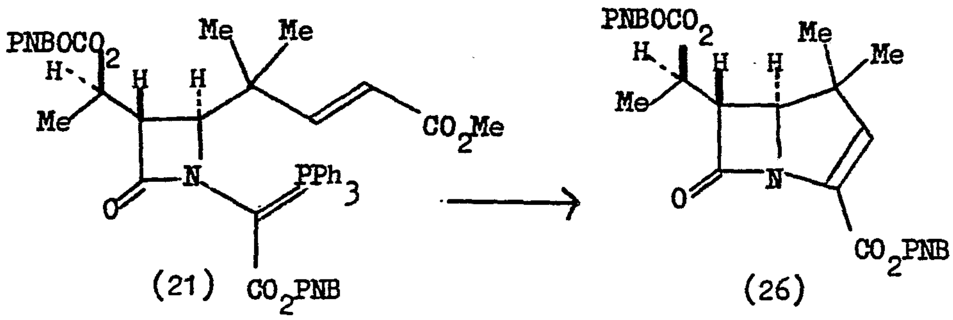

(21) (26)

A solution of p-nitrobenzyl 2-[(3RS,4RS)-4-[(E)-1,1-dimethyl-3-methoxycarbonylprop-2-enyl]-3-[(1SR)-1-(p-nitrobenzyloxycarbonyloxy)-ethyl]-2-oxoazetidin-1-yl]-2-(triphenylphosphoranylidene)acetate (21) (0.100 g) in ethyl acetate (10 ml) was cooled to 0°C and treated with trifluoroacetic acid (0.3 ml). It was stirred for 10 min and then cooled to -70°C. A stream of ozone was bubbled through the solution until it just became blue, and excess ozone was then blown off with argon. An ethyl acetate solution of triphenylphosphine (0.030 g) was added and after 0.5 h the solution was allowed to warm to 0°C. Saturated aqueous sodium hydrogen carbonate (10 ml) was added with vigorous stirring, which was continued for 5 min. The organic phase was then separated, washed with brine, dried over sodium sulphate, and kept overnight at room temperature. The solution was concentrated and chromatographed on silica gel 60 (230 - 400 mesh ASTM) eluting with ethyl acetate/hexane 3:7 grading to 7:3 to give p-nitrobenzyl (5RS,6RS)-4,4-dimethyl-6-[(1SR)-1-(p-nitrobenzyloxycarbonyloxy)ethyl]-7-oxo-1-azabicyclo[3.2.0]hept-2-en-2-carboxylate (26) (0.015 g) as a colourless gum; $\lambda_{max.}$ (EtOH) 263 nm ($\epsilon$ 24,000); $\nu_{max.}$ ($CHCl_3$) 3050, 2970, 2870, 1780, 1740, 1610 and 1525 $cm^{-1}$; $\delta$ ($CDCl_3$) 1.16 and 1.32 (6H, 2s, 4-$(CH_3)_2$), 1.48 (3H, d, J 6Hz, $CH_3$-C-O), 3.45 (1H, dd, J 6 and 3 Hz, 6-H), 3.88 (1H, d, J 3Hz, 5-H), 5.19 (1H, quin, J 6Hz, -CH-O), 5.27 (2H, s, benzylic $CH_2$), 5.28 and 5.44 (2H, ABq, J 13 Hz, benzylic $CH_2$), 6.36 (1H, s, 3-H), 7.55 (2H, d, J 9 Hz, aromatic),

7.61 (2H, d, _J_ 9Hz, aromatic), and 8.24 (4H, d, _J_ 9Hz, aromatic) (Found: _M_ $^+$, 539.155 5. $C_{26}H_{25}N_3O_{10}$ requires _M_, 539. 153 9).

Example 22

Sodium (5RS,6RS)-4,4-dimethyl-6-[(1SR)-1-hydroxyethyl]-3-(pyrimidin-2-ylsulphinyl)-7-oxo-1-azabicyclo[3.2.0]hept-2-en-2-carboxylate

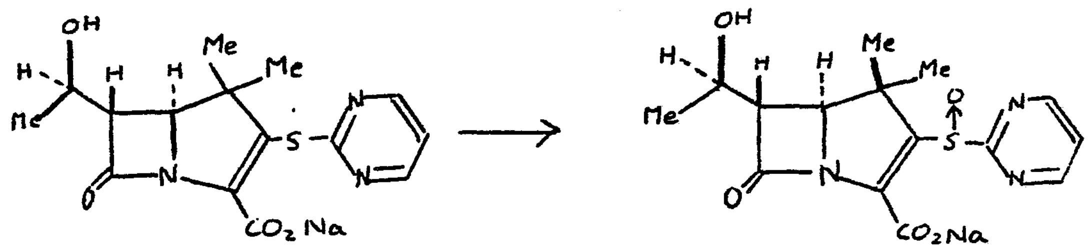

(25) (27)

Sodium (5RS,6RS)-4,4-dimethyl-6-[(1SR)-1-hydroxyethyl]-3-(pyrimidin-2-ylthio)-7-oxo-1-azabicyclo[3.2.0]hept-2-en-2-carboxylate (25) (0.006 g) in water (6 ml) was treated with m-chloroperbenzoic acid (0.0032 g), for 15 minutes. The reaction mixture was filtered and adjusted to pH 7 by the addition of saturated aqueous sodium hydrogen carbonate solution to afford the title compound (27); $\lambda_{max.}$ ($H_2O$) 232 and 278 nm.

Example 23

p-Nitrobenzyl(5RS,6RS)-4,4-dimethyl-6-[1SR)-1-(p-nitrobenzyloxycarbonyloxy)ethyl]-3-(pyrimidin-2-ylsulphinyl) 7-oxo-1-azabicyclo[3.2.0]hept-2-en-2-carboxylate

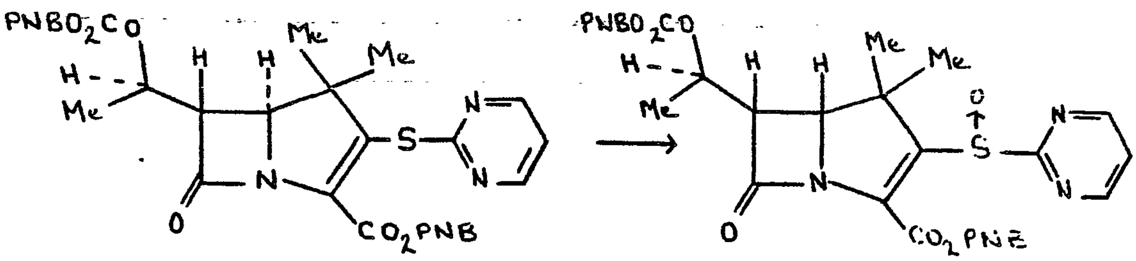

(24) (28)

p-Nitrobenzyl (5RS,6RS)-4,4-dimethyl-6-[(1SR)-1-(p-nitrobenzyloxycarbonyloxy)ethyl]-3-(pyrimidin-2-ylthio)-7-oxo-1-azabicyclo[3.2.0]-hept-2-en-2-carboxylate (24) (0.116 g) in dichloromethane was cooled to 0°C. m-Chloroperbenzoic acid (0.044 g of 85% material) in dichloromethane (1 ml) was added and the mixture was stirred at 0°C for 4 h. The mixture was diluted to 10 ml with dichloromethane and extracted with saturated aqueous sodium hydrogen carbonate solution (2 x 2ml). These extracts were combined and back-extracted with dichloromethane (1 ml). The combined organic phases were washed with brine (2 x 2 ml) and dried (sodium sulphate). Evaporation gave an oil, which was chromatographed silica gel 60 (Merck 9385, 230-400 mesh ASTM; 12x2 cm). Elution with ethyl acetate-hexane (3:1) (4 ml fractions) afforded unreacted p-nitrobenzyl (5RS, 6RS)-4,4-dimethyl-6-[(1SR)-1-(p-nitrobenzyloxycarbonyloxy)ethyl]-3-(pyrimidin-2-ylthio)-7-oxo-1-azabicyclo[3.2.0]-hept-2-en-2-carboxylate (24) (0.025g) Continued elution of the column with ethyl acetate gave a band which contained both sulphoxide isomers of p-nitrobenzyl (5RS,6RS)-4,4-dimethyl-6-[(1SR)-1-(p-nitrobenzyloxycarbonyloxy)ethyl]-3-(pyrimidin-2-yl-

sulphinyl)-7-oxo-1-azabicyclo[3.2.0]hept-2-en-2-carboxylate (28) (1:1 ratio by t.l.c. analysis and n.m.r. spectrum) (0.069 g). Ending fractions from the band gave the least polar sulphoxide (Isomer A) as a colourless gum; $\lambda_{max}$ (EtOH) 320infl and 265 nm; $\nu_{max}$ ($CHCl_3$) 1790, 1745, 1710sh, 1610, 1560, 1525, 1380 and 1350 $cm^{-1}$. Trailing fractions from the band contained the pure sulphoxide epimer (Isomer B), a colourless gum; $\lambda_{max}$ (EtOH) 321infl and 262 nm; $\nu_{max}$ ($CHCl_3$) 1790, 1740, 1705sh, 1610, 1560, 1525, 1380 and 1350 $cm^{-1}$.

Example 24

p-Nitrobenzyl (5RS,6RS)-3-[(Z)-2-carbamoylethenylthio]-4,4-dimethyl-6-[(1SR)-1-(p-nitrobenzyloxycarbonyloxy)-ethyl]-7-oxo-1-azabicyclo[3.2.0]hept-2-en-2-carboxylate

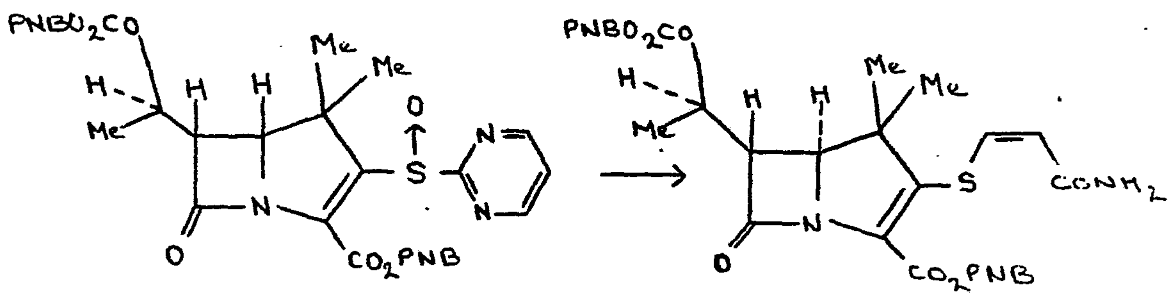

(28) (29)

To (Z)-2-carbamoylethenylthio acetate (0.011 g) in dioxan (0.5 ml) was added 0.1M aqueous sodium hydroxide solution (1.5 ml). After stirring at room temperature for 15 mins the solvents were removed *in vacuo* and replaced with water (0.5 ml).

p-Nitrobenzyl (5RS,6RS)-4,4-dimethyl-6-[(1SR)-1-(p-nitrobenzyloxycarbonyloxy)ethyl]-3-(pyrimidin-2-yl-sulphinyl)-7-oxo-1-azabicyclo[3.2.0]hept-2-en-2-carboxylate (28) (1:1 ratio of Isomers A and B) (0.050 g) in

dichloromethane (3 ml) was cooled to 0°C and cetyltriethylammonium chloride (2 mg of a preparation containing 50% water) was added. To this solution was then added the aqueous sodium (Z)-2-carbamoylethenylthio acetate solution prepared above. Vigorous stirring was maintained for 4h. The mixture was diluted with dichloromethane (10 ml). The organic phase separated, washed with brine, and dried (sodium sulphate). Evaporation gave an oil which was chromatographed on silica gel 60 (Merck 9385, 230-400 mesh ASTM; 3 x 1 cm). Elution with ethylacetate (2 ml fractions) gave (fractions 5, 6) p-nitrobenzyl (5RS, 6RS)-3-[(Z)-2-carbamoylethenylthio]-4,4-dimethyl-6-[(1SR)-1-(p-nitrobenzyloxycarbonyloxy)ethyl]-7-oxo-1-azabicyclo[3.2.0]-hept-2-en-2-carboxylate (29) as an amorphous solid (0.008 mg); $\lambda_{max}$ (EtOH) 320 nm. Later fractions (8-12) gave unreacted p-nitrobenzyl (5RS,6RS)-4,4-dimethyl-6-[(1SR)-1-(p-nitrobenzyloxycarbonyloxy)ethyl]-3-(pyrimidin-2-ylsulphinyl)-7-oxo-1-azabicyclo[3.2.0]hept-2-en-2-carboxylate (28) (IsomerA) (0.018 g).

Example 25

Sodium(5RS,6RS)-3-[(Z)-2-acetamidoethenylthio]-4,4-dimethyl-6-[(1SR)-1-hydroxyethyl]-7-oxo-1-azabicyclo [3.2.0]-hept-2-en-2-carboxylate

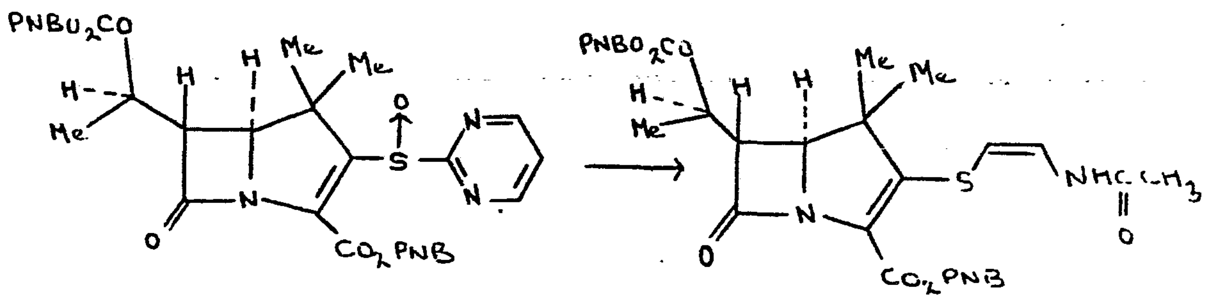

(24) (31)

p-Nitrobenzyl (5RS,6RS)-4,4-dimethyl-6-[(1SR)-1-(p-nitrobenzyloxycarbonyloxy)ethyl]-3-(pyrimidin-2-yl-sulphinyl)-7-oxo-1-azabicyclo[3.2.0]hept-2-en-2-carboxylate (24) and sodium (Z)-2-acetamidoethenylthiolate were converted to p-nitrobenzyl (5RS,6RS)-3-[(Z)-2-acetamidoethenylthio]-4,4-dimethyl-6-[(1SR)-1-(p-nitrobenzyloxycarbonyloxy)ethyl]-7-oxo-1-azabicyclo[3.2.0]-hept-2-en-2-carboxylate (31) by the method described in Example 24. $\nu_{max}$ ($CHCl_3$) 1755, 1710, 1610, 1530, 1350 $cm^{-1}$, $\lambda_{max}$ (EtOH) 325 nm. The p-nitrobenzyl ester is hydrogenated to the sodium salt by the method described in Example 26.

0060077

Example 26

Sodium (5RS,6RS)-3-[(Z)-2-carbamoylethenylthio]-4,4-dimethyl-6-[(1SR)-1-hydroxyethyl]-7-oxo-1-azabicyclo[3.2.0]hept-2-en-2-carboxylate

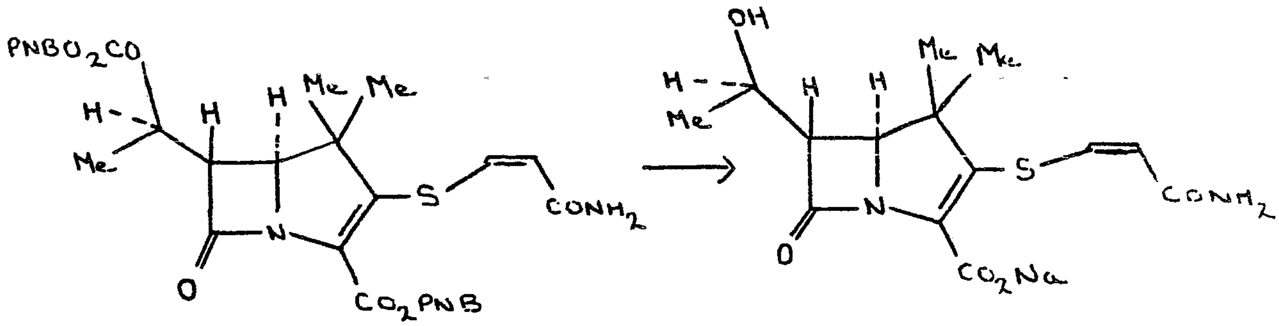

(29) (32)

A suspension of 10% palladium-carbon catalyst (0.010 g) in dioxan-water (2:1) (1 ml) was shaken in an atmosphere of hydrogen gas for 0.5h. To this was added p-nitrobenzyl (5RS,6RS)-3-[(Z)-2-carbamoylethenylthio]-4,4-dimethyl-6-[(1SR)-1-(p-nitrobenzyloxycarbonyloxy)-ethyl]-7-oxo-1-azabicyclo[3.2.0]-hept-2-en-2-carboxylate (29) (0.008 g) as a suspension in dioxan (2 ml) containing 0.05M phosphate buffer (0.3 ml). The suspension was shaken vigorously in an atmosphere of hydrogen gas for 2.5 h. Sodium hydrogen carbonate (0.002 g) in water (1 ml) was added, the suspension shaken vigorously, and then filtered through celite, washing with dioxan and water. The combined filtrate and washings were evaporated in vacuo to give an aqueous solution, which was extracted with ethyl acetate (3 x 1 ml). The aqueous solution was then concentrated in vacuo to 1 ml, and chromatographed on "Biogel P2", eluting with water (2 ml fractions). Fractions 5-7 afforded sodium (5RS,6RS)-3-[(Z)-2-carbamoylethenylthio]-4,4-dimethyl-6-[(1SR)-1-hydroxyethyl]-7-oxo-1-azabicyclo[3.2.0]hept-2-en-2-carboxylate (32) (0.002 g); $\lambda_{max}$ (EtOH) 302 nm.

Example 27

1,1-Bis(hydroxymethyl)cyclopropane

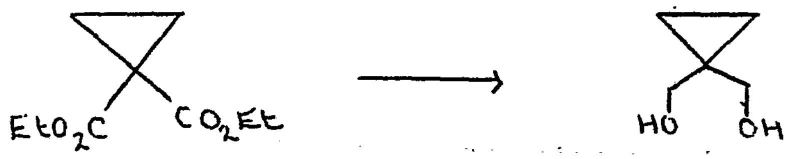

(33) (34)

Diethyl 1,1-cyclopropanedicarboxylate (33) (25 g) in dry ether (50 ml) was added in a dropwise fashion to a stirred suspension of lithium aluminium hydride (6.65g) in diethyl ether (150 ml), such that a gentle reflux was maintained. After complete addition the reaction mixture was heated under reflux for a further 4 hours. Aqueous hydrochloric acid solution (5M) was added cautiously until all effervescence ceased.

The total two phase system was filtered through celite and then extracted continuously with ether for 12hr. Drying ($MgSO_4$) and removal of solvent gave the crude product 1,1-bis(hydroxymethyl)cyclopropane (34), as a pale yellow oil (11.38 g); $\nu_{max}$ ($CHCl_3$) 3325, 2925 and 2875 $cm^{-1}$; $\delta$ ($CDCl_3$), 0.50 (4H, s, cyclopropane ring $-CH_2-$), 3.62 (4H, s, 2 x side-chain $-CH_2-$), 4.63 (2H, s, -OH, $D_2O$ exchangeable).

Example 28

1-Bromomethyl-1-hydroxymethylcyclopropane

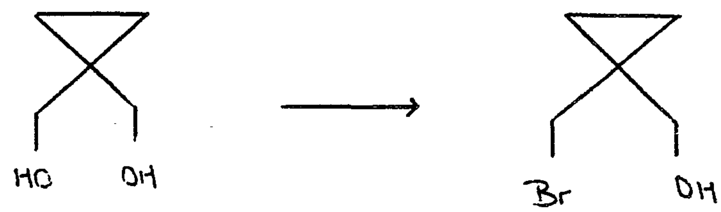

(34) (35)

1,1-Bis(hydroxymethyl)cyclopropane (34), (11.31g) in dry tetrahydrofuran (400 ml) was cooled to 0°C. To the stirred solution was added carbon tetrabromide (36.87g), followed by the dropwise addition of triphenylphosphine (29.07g) in tetrahydrofuran (400 ml) over 30 mins. After stirring at room temperature for 3h, a white precipitate had formed. T.l.c. analysis (elution with ethyl acetate) on silica plates showed formation of product together with triphenylphosphine oxide. Filtration and removal of the solvent gave a yellow gum which was chromatographed on silica gel (Merck, Art. 7729; 230 mesh ASTM) (eluting with ethyl acetate/hexane 1:1). The product, a straw-coloured oil, was 1-bromomethyl-1-hydroxymethylcyclopropane (35); δ ($CDCl_3$) 0.70 (4H, m, cyclopropane ring $-C\underline{H}_2-$), 2.97 (1H, s, -OH, $D_2O$ exchangeable), 3.50 (2H, s, $-C\underline{H}_2Br(OH)$), 3.56 (2H, s, $-C\underline{H}_2OH(Br)$).

Example 29

1-Azidomethyl-1-hydroxymethylcyclopropane

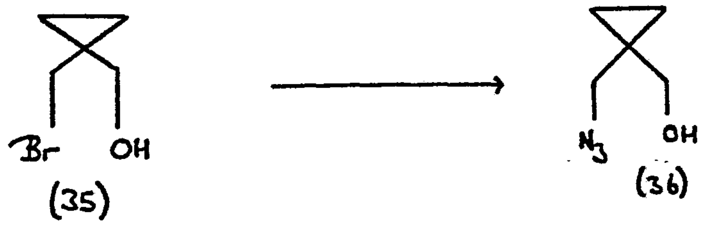

(35) (36)

1-Bromomethyl-1-hydroxymethylcyclopropane (35) from example 2, in dry dimethylformamide (200 ml) was heated with stirring, with sodium azide (7.2 g) and tetramethylguanidinium azide (1.75 g) at 90°C for 12h. The dimethylformamide was removed in vacuo and the residue was diluted with ethyl acetate and washed with portions of brine. Drying, evaporation, and chromatography of the residue on silica gel (Merck, Art 7729 230 Mesh ASTM) (eluting with 1:1, ethyl acetate/hexane) gave 1-azidomethyl-1-hydroxymethylcyclopropane (36) as a pale yellow oil. (5.41g); $\nu_{max}$ ($CHCl_3$), 3600 (sharp), 3440 (broad), and 2100 (strong) $cm^{-1}$; $\delta$ ($CDCl_3$), 0.58 (4H, s, cyclopropane ring $-C\underline{H}_2-$), 2.01 (1H, s, -OH, $D_2O$ exchangeable) 3.34 (2H, s, $-C\underline{H}_2N_3$), 3.56 (2H, s, $-C\underline{H}_2OH$).

Example 30

1-[5-Oxa-12-azadispiro[2.2.5.2]tridecan-12-yl]butane-1,3-dione (39)

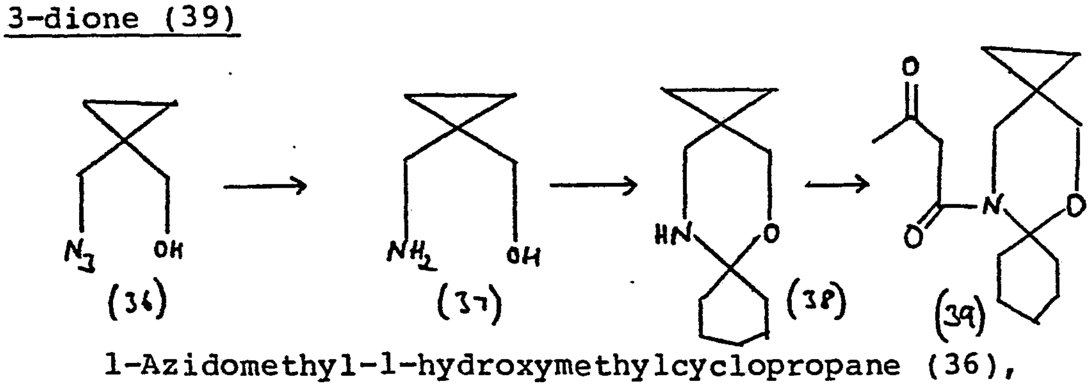

(36) (37) (38) (39)

1-Azidomethyl-1-hydroxymethylcyclopropane (36), (5.36 g) in ethanol (120 ml) containing 10% palladium/charcoal catalyst (245 mg) was shaken in an

atmosphere of hydrogen gas for 1h. The hydrogen/nitrogen mixture was then removed in vacuo and replaced with fresh hydrogen. Hydrogenation for a further 1h provided a sample which was shown by infra red spectroscopy to contain no azido-compound (36). Filtration through celite and evaporation of ethanol gave 1-aminomethyl-1-hydroxymethylcyclopropane (37) as a yellow gum. This was taken up in dry benzene (245 ml) and cyclohexanone (4.13 g) was added. The mixture was heated under reflux, with removal of water under azeotropic conditions, for 20h. Evaporation gave 3,3-spirocyclopropyl-1-oxa-5-azaspiro[5.5]undecane (38). This product was redissolved in dry benzene (180 ml) and cooled to 0°C. Diketene (3.3 ml) was added, and the reaction maintained at 0°C for 1h, and at room temperature for a further 2h. The solvent was removed and the residue chromatographed on silica gel (Merck, Art. 7729, 230 mesh ASTM) eluting with dichloromethane - ethyl acetate mixtures (gradient elution). The product, 1-[5-Oxa-12-azadispiro[2.2.5.2]tridecan-12-yl]butane-1,3-dione (39) was collected as a pale yellow gum (6.42 g);

$\nu_{max}$ ($CHCl_3$), 1720 and 1635 $cm^{-1}$;

$\delta$($CDCl_3$) 0.46 (2H, m, cyclopropyl-C$\underline{H}_2$-), 0.62 (2H, m, cyclopropyl-C$\underline{H}_2$-), 1.26-1.63 (6H, complex m, 3 x cyclohexyl-C$\underline{H}_2$-), 1.87 (2H, m, cyclohexyl-C$\underline{H}_2$-), 2.26 (3H, s, -C$\underline{H}_3$CO-), 2.72 (2H, dt, $\underline{J}$ 4, 13Hz; cyclohexyl -C$\underline{H}_2$-), 3.22 (2H, s, -NC$\underline{H}_2$-), 3.41 (2H, s, -OC$\underline{H}_2$-), 3.67 (2H, s, MeCOC$\underline{H}_2$-). (Found: $\underline{M}^+$, 265.1698, $C_{15}H_{23}NO_2$ requires $\underline{M}$, 265.1676).

Example 31

2-Diazo-1-[5-oxa-12-azadispiro[2.2.5.2]tridecan-12-yl]-butane-1,3-dione (40)

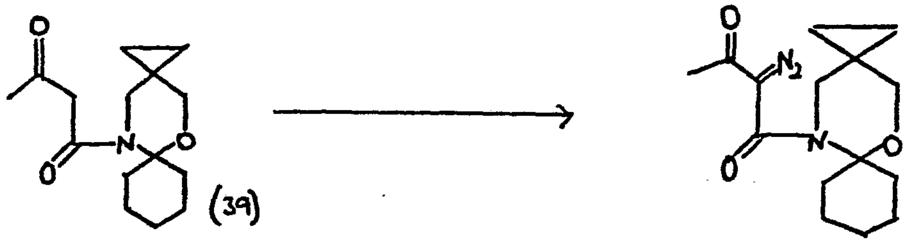

(39) (40)

1-[5-Oxa-12-azadispiro[2.2.5.2]tridecan-12-yl]-butane-1,3-dione (39),(6.39 g) in dry benzene (230 ml) was cooled to 0°C in ice and treated with triethylamine (6.72 ml), methanesulphonylazide (25.4 ml; of a toluene solution containing 0.230 g/ml of reagent), and piperidine (0.5 ml). After 1h at 0°C and 12h at room temperature the solution was evaporated and the residue was chromatographed on silica gel (Merck Art. 7729; 230 mesh ASTM) (eluting with ethyl acetate/hexane 3:10 grading to 1:1). The yellow, oily product readily crystallised (ethyl acetate/hexane) to give 2-Diazo-1-[5-oxa-12-azadispiro[2.2.5.2]tridecan-12-yl]-butane-1,3-dione (40) as a yellow crystalline solid (3.3 g, 47%), mp 108-114°C (decomp);

$\nu_{max}$ ($CHCl_3$) 2110, and 1630 $cm^{-1}$;

$\lambda_{max}$ (EtOH) 217.9 nm ($\mathcal{E}$, 61,240);

$\delta$($CDCl_3$) 0.53 and 0.62 (4H, t, J 5Hz; $A_2B_2$; cyclopropyl $-CH_2CH_2-$), 1.27-1.64 (6H, m, cyclohexyl $-(CH_2)_3-$), 1.95 (2H, d, J 13Hz, 2 x -CH- cyclohexyl-), 2.32 (3H, s, $CH_3CO-$), 2.63 (2H, dt, J 13, 4Hz, 2 x -CH-cyclohexyl-), 3.42 (2H, s, $-NCH_2-$), 3.70 (2H,s, $-OCH_2-$) (Found: M - $N_2^+$, 263.1536, $C_{15}H_{21}NO_3$ requires m/z 263.1522).

Example 32

(6'RS, 7'RS)-7'-Acetyldispiro[cyclohexane-1,2'-[3]oxa[1]azabicyclo[4.2.0]octane-5',1"-cyclopropan]-8'-one (41)

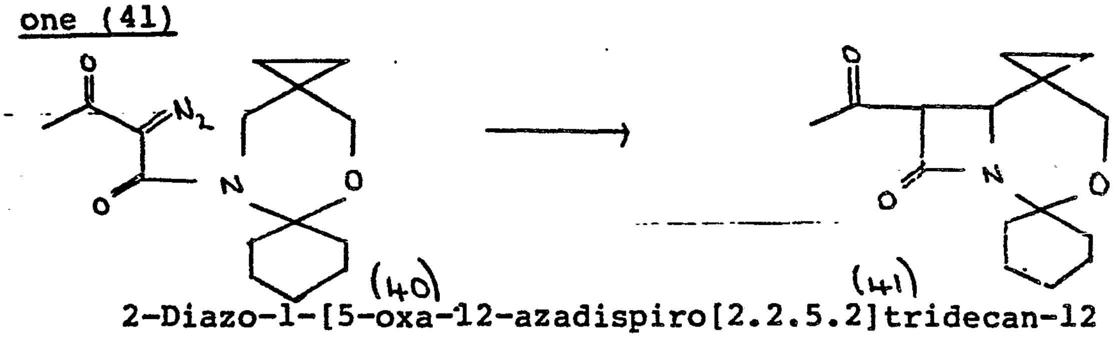

(40) (41)

2-Diazo-1-[5-oxa-12-azadispiro[2.2.5.2]tridecan-12-yl]-butane-1,3-dione (40), (3.3 g) in dry benzene (30 ml) and rhodium (II) acetate (0.48 g) was maintained at 60°C for 24h. The reaction mixture was filtered through celite and concentrated. The residue was chromatographed on silica gel (Merck Art. 7729; 230 mesh ASTM) eluting with ethyl acetate/hexane (3:10). A colourless oil was obtained, which readily crystallised to give (6'RS,7'RS)-7'-Acetyldispiro[cyclohexane-1,2'-[3]oxa[1]azabicyclo[4.2.0]octane-5',1"-cyclopropan]-8'-one (41), (1.97 g), mp 107-108°C (ex ethyl acetate/hexane; white needles);

$\nu_{max}$ ($CHCl_3$) 1755 and 1715 $cm^{-1}$;

$\delta$($CDCl_3$) 0.48-0.74 (4H, m, cyclopropyl $-(C\underline{H}_2)_2-$), 1.43-1.89 (10H, m, cyclohexyl $-(C\underline{H}_2)_5-$), 2.30 (3H, s, $C\underline{H}_3CO-$), 3.18 (1H, d, $\underline{J}$ 12Hz; $-C\underline{H}-O-$), 3.70 (1H, d, $\underline{J}$ 2Hz; C6'-H), 3.92 (1H, dd, $\underline{J}$ 2, 12Hz; $-C\underline{H}-O-$), 4.16 (1H, d, $\underline{J}$ 2Hz; C7';H) (Found: $\underline{M}^+$, 263.1524; $C_{15}H_{21}NO_3$ requires $\underline{M}$, 263.1519).

Example 33

(6'RS,7'RS)-7'-[1SR)-1-hydroxyethyl]-dispiro[cyclohexane-1,2'-[3]oxa[1]azabicyclo-[4.2.0]octane-5',1"cyclo-propan]-8'-one (42)

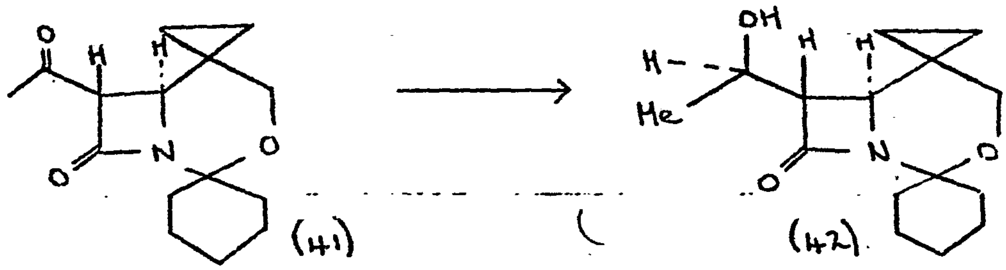

(41)

(42)

(6'RS, 7'RS)-7'-Acetyldispiro[cyclohexane-1,2'-[3] oxa[1]azabicyclo[4.2.0]octane-5',1"-cyclopropan]-8'-one (41), (1.95 g) in dry tetrahydrofuran (20 ml) was cooled to 0°C in ice and treated with an excess of potassium tri-sec-butylborohydride (14.8 ml of a 1M solution in tetrahydrofuran). After initial effervescence had subsided a further 10 ml of tetrahydrofuran was added and the reaction mixture was allowed to warm to room temperature. The reaction was quenched by the addition of saturated aqueous ammonium chloride solution and, after extraction with portions of ethyl acetate, a gum was obtained. Chromatography on silica gel (Merck Art. 7729; 230 mesh ASTM) eluting with ethyl acetate/hexane (1:1) grading to ethyl acetate gave (6'RS,7'RS)-7'-[1SR)-1-hydroxyethyl]-dispiro[cyclohexane-1,2'-[3]oxa[1]azabicyclo-[4.2.0]octane-5',1"cyclo-propan]-8'-one (42) (0.290 g) as a colourless gum;

$\nu_{max}$ ($CHCl_3$) 3600-3100 (Broad) and 1740 $cm^{-1}$.

Example 34

(6'RS,7'RS)-7'-[(1SR)-1-(p-nitrobenzyloxycarbonyloxy)-ethyl]dispiro[cyclohexane 1,2'-[3]oxa[1]azabicyclo-[4.2.0]octane-5'-1"-cyclopropan]-8'-one (43)

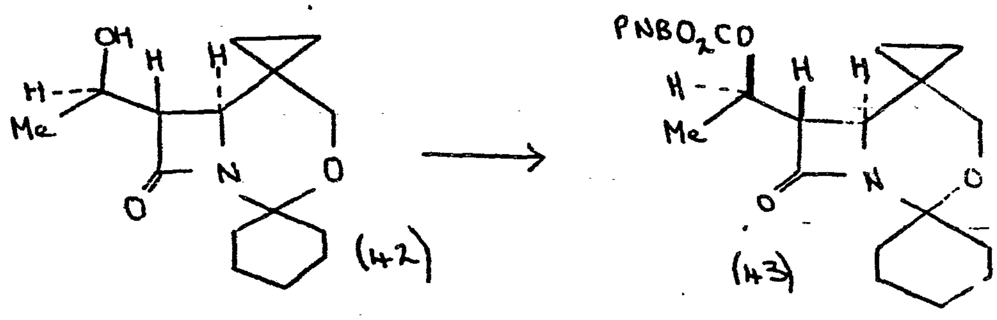

(42) (43)

(6'RS,7'RS)-7'-[1SR)-1-hydroxyethyl]dispiro[cyclohexane-1,2'-[3]oxa[1]azabicyclo[4.2.0]octane-5',1"-cyclopropan]-8'-one (42) (0.280 g) in dry tetrahydrofuran (20 ml) was cooled to -70°C and treated with m-butyllithium (0.75 ml, of a 1.55 M solution in hexane). After 15 min, p-nitrobenzyl chloroformate (0.32 g) in tetrahydrofuran (5 ml) was added dropwise. After allowing to warm to room temperature, and stirring for 2h, saturated ammonium chloride was added. Extraction with ethyl acetate, drying and removal of solvent afforded a brown gum. This was chromatographed on silica gel (Merck Art. 7729; 230 mesh ASTM) eluting with ethyl actate/hexane (1:1). (6'RS,7'RS)-7'-[(1SR)-1-(p-nitrobenzyloxycarbonyloxy)-ethyl]dispiro[cyclohexane 1,2'-[3]oxa[1]azabicyclo-[4.2.0]octane-5'-1"-cyclopropan]-8'-one (43) was obtained as a gum (0.088 g);

$\nu_{max}$ ($CHCl_3$), 1750, 1610 (weak), 1525, 1450, 1380 and 1350 $cm^{-1}$.

Example 35

(3RS,4RS)-4-[1-(hydroxymethyl)cyclopropyl]-3-[(1SR)-1-(p-nitrobenzyloxycarbonyloxy)ethyl]azetidin-2-one (44)

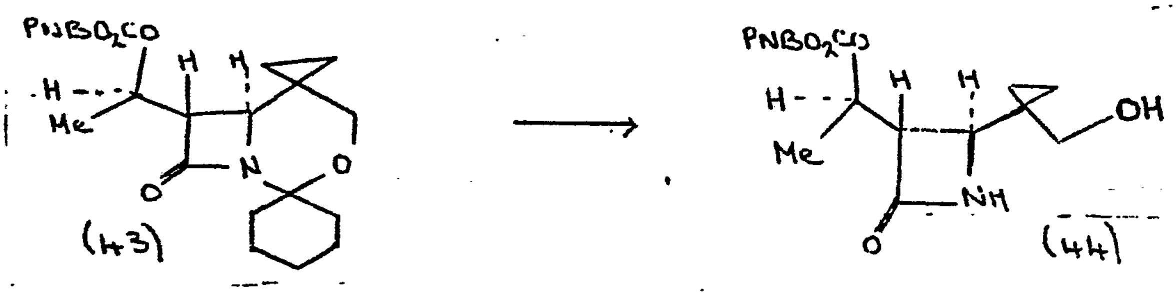

(43) (44)

(6'RS,7'RS)-7'-[(1SR)-1-(p-nitrobenzyloxycarbonyl-oxy)-ethyl]dispiro[cyclohexane 1,2'-[3]oxa[1]-azabicyclo-[4.2.0]octane-5'-1"-cyclopropan]-8'-one (43) (0.084 g) in dioxan (5 ml) was treated with dilute aqueous sulphuric acid (0.75 ml, 10% solution) at 75°C for 3h. Saturated aqueous sodium hydrogen carbonate was added cautiously until the acid was neutralised. The dioxan was removed *in vacuo* and the aqueous phase extracted with ethyl acetate (3 x 20 ml). Chromatography on silica gel (Merck Art. 7729; 230 mesh ASTM), eluting with ethyl acetate afforded (3RS,4RS)-4-[1-(hydroxymethyl)cyclopropyl]-3-[(1SR)-1-(p-nitrobenzyloxycarbonyloxy)ethyl]azetidin-2-one (44) as a colourless gum (0.033 g);

$\nu_{max}$ ($CHCl_3$) 3600-3150 br, 3420, 1760, 1740 sh, 1610, 1525, 1375, and 1350 $cm^{-1}$;

$\delta$ ($CDCl_3$) 0.50 (4H, s, cyclopropyl $-(C\underline{H}_2)_2-$), 1.44 (3H, d, $\underline{J}$ 6Hz; $-C\underline{H}_3$), 2.42 br (1H, s, $-O\underline{H}$), 3.06-3.80 (4H, m, $-C\underline{H}_2OH$, C4-H, C3-H), 5.12 (1H, m, $-C\underline{H}-$ side chain), 5.28 (2H, s, $-OCO_2C\underline{H}_2-$), 6.53 br (1H, s, $-N\underline{H}$), 7.56 and 8.26 (4H, d, $\underline{J}$ 9Hz; aom-$\underline{H}$).

Example 36

Methyl(E)-3-[1-[(3RS,4RS)-3-[(1SR)-1-(p-nitrobenzyloxycarbonyloxy)ethyl]-2-oxoazetidin-4-yl]cyclopropyl]prop-2-enoate (45).

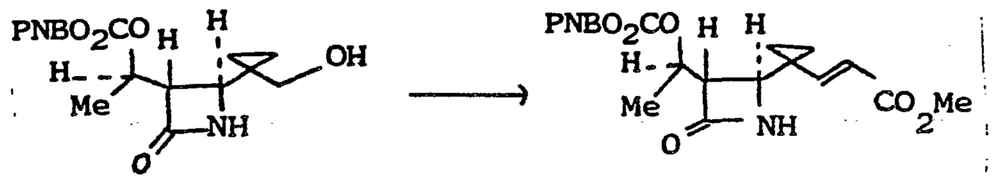

(44) (45)

(3RS,4RS)-4-[1-(hydroxymethyl)cyclopropyl]-3-[(1SR)-1-(p-nitrobenzyloxycarbonyloxy)ethyl]-azetidin-2-one (44) (0.033 g) in dry dichloromethane (3 ml) at 35°C was treated with pyridinium dichromate (0.047 g) for 3h. Filtration through celite, dilution with ethyl acetate, washing with citric acid and brine afforded a solution of the intermediate aldehyde. This wa dried and concentrated to 3 ml. To this solution was added carbomethoxymethylenetriphenylphsophorane (0.032 g). After stirring at room temperature for 48h and at 70°C for 4h, chromatography of the residue on silica gel (Merck Art. 7729; 230 mesh ASTM) eluting with ethyl acetate/hexane (7:10), afforded as a colourless foam, Methyl(E)-3-[1-[(3RS,4RS)-3-[(1SR)-1-(p-nitrobenzyloxycarbonyloxy)ethyl]-2-oxoazetidin-4-yl]cyclopropyl]prop-2-enoate (45), (0.020 g);

$\nu_{max}$ ($CHCl_3$) 3420, 1765, 1720, 1645, 1610, 1525 and 1350 $cm^{-1}$.

Example 37

p-Nitrobenzyl(5RS,6RS)-6-[(1SR)-1-(p-nitrobenzyloxy-carbonyloxy)ethyl]-3-(pyrimidin-2-ylthio)-7-oxospiro-[[1]azabicyclo[3.2.0]hept[2]ene-4,1'-cyclopropane]-2-carboxylate (48)

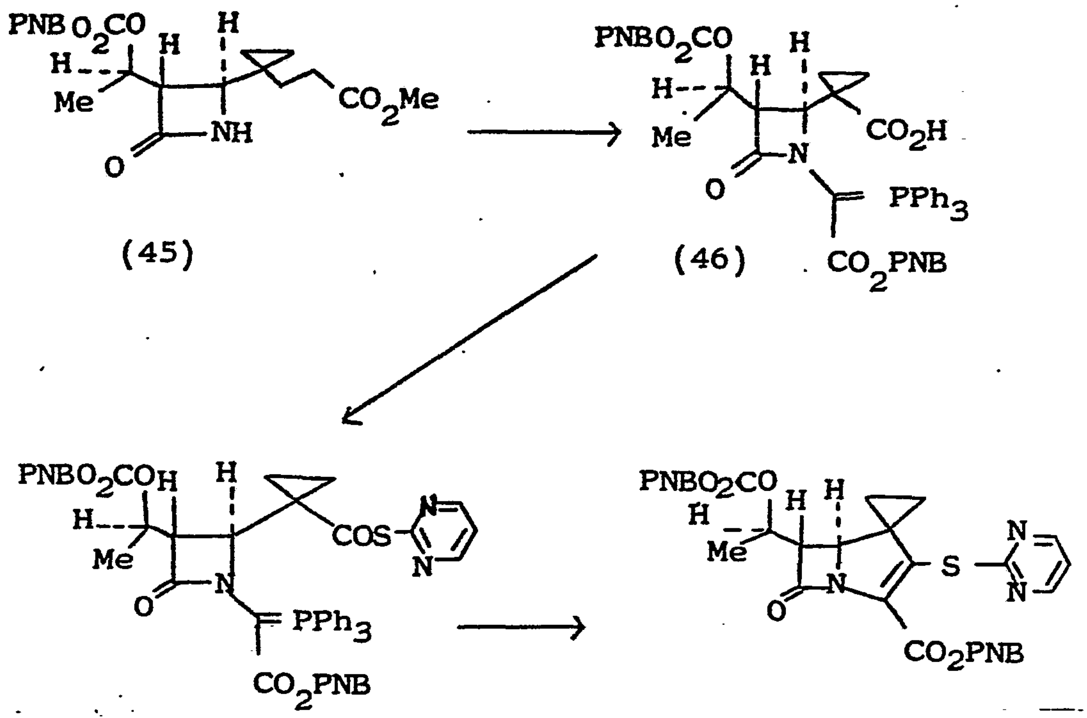

(45) (46)

(47) (48)

Methyl(E)-3-[1-[(3RS,4RS)-3-[(1SR)-1-(p-nitrobenzyloxycarbonyloxy)ethyl]-2-oxoazetidin-4-yl] cyclopropyl]prop-2-enoate (45) was converted via p-nitro-benzyl 2-[(3Rs,4RS)-4-(1-carboxycyclo-propan-1-yl)-3-[(1SR)-1-(p-nitrobenzyloxycarbonyl-oxy)ethyl]-2-oxoazetidin-1-yl]-2-(triphenyl-phosphoranylidene)- acetate (46), and p-nitrobenzyl 2-[(3RS,4RS)-3-[(1SR)-1-(p-nitrobenzyloxycarbonyloxy)-ethyl]-2-oxo-4-[1-(pyrimidin-2-ylthiocarbonyl)-cyclopropan-1-yl]-azetidin-1-yl]-2-(triphenyl-phosphoranylidene)acetate (47), and thence to p-nitrobenzyl(5RS,6RS)-6-[(1SR)-1-(p-nitrobenzyloxycarbonyloxy)ethyl]-3-(pyrimidin-2-ylthio)-7-oxospiro[[1]azabicyclo[3.2.0]hept[2]ene-4,1'-cyclopropane]-2-carboxylate (48), by the methods described in Examples 15 to 20.

Example 38

Sodium (5RS,6RS)-6-[(1SR)-1-hydroxyethyl]-3-(pyrimidin-2-ylthio)-7-oxospiro[[1]azabicyclo[3.2.0]hept[2]ene-4,1'-cyclopropane]-2-carboxylate (49)

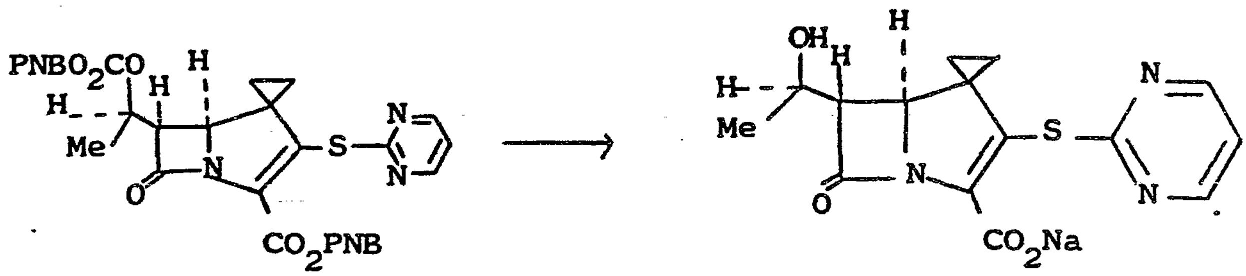

(48) (49)

p-Nitrobenzyl(5RS,6RS)-6-[(1SR)-1-(p-nitrobenzyloxy-carbonyloxy)ethyl]-3-(pyrimidin-2-ylthio)-7-oxospiro-[[1]azabicyclo[3.2.0]hept[2]ene-4,1'-cyclopropane]-2-carboxylate (48) was hydrogenolysed under the conditions described in Example 20 to give Sodium (5RS,6RS)-6-[(1SR)-1-hydroxyethyl]-3-(pyrimidin-2-ylthio)-7-oxospiro[[1]azabicyclo[3.2.0]hept[2]ene-4,1'-cyclopropane]-2-carboxylate (49)

Example 39

Sodium (5RS,6RS)-6-[(1SR)-1-hydroxyethyl]-3-(pyrimidin-2-ylsulphinyl)-7-oxospiro[[1]azabicyclo[3.2.0]hept[2]-ene-4,1'-cyclopropane]-2-carboxylate (50)

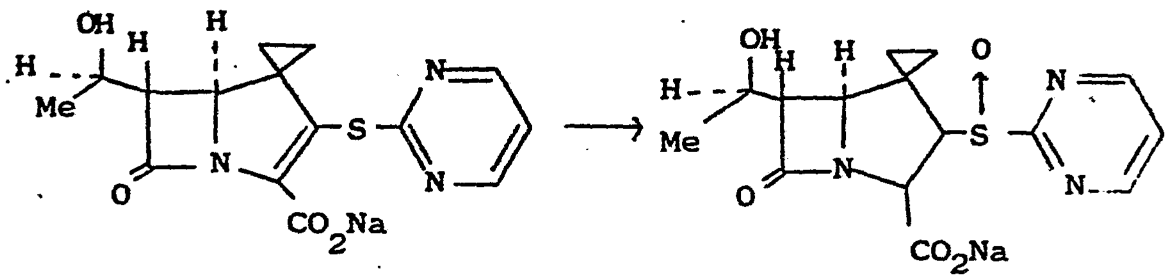

(49) (50)

Sodium (5RS,6RS)-6-[(1SR)-1-hydroxyethyl]-3-(pyrimidin-2-ylthio)-7-oxospiro[[1]azabicyclo[3.2.0]-hept[2]ene-4,1'-cyclopropane]-2-carboxylate (49) in dichloromethane was oxidised with m-chloroperbenzoic acid as described in Example 22, to give Sodium (5RS,6RS)-6-[(1SR)-1-hydroxyethyl]-3-(pyrimidin-2-ylsulphinyl)-7-oxospiro[[1]azabicyclo[3.2.0]hept[2]-ene-4,1'-cyclopropane]-2-carboxylate (50)

MIC DATA (Broth) For sodium (5RS,6RS)-3-[(Z)-2-carbamoylethenylthio]-4,4-dimethyl-6-[(1SR)-1-hydroxyethyl]-7-oxo-1-azabicyclo[3.2.0]hept-2-en-2-carboxylate (32)

| Organism | MIC (µg/ml) |
|---|---|
| C. freundii E8 | 25 |
| E. cloacae N1 | 25 |
| E. coli ESS | 3.1 |
| E. coli 0111 | 50 |
| E. coli JT39 | 12.5 |
| K. aerogenes A | 6.2 |
| K. aerogenes Ba95 | 50 |
| P. mirabilis 977 | 25 |
| P. morganii 1580 | 25 |
| P. rettgeri Wm16 | 25 |
| P. vulgaris W091 | 25 |
| S. typhimurium CT10 | 50 |
| S. sonnei MB11 1967 | 25 |
| B. subtilis A | 1.6 |
| S. aureus Oxford | 6.2 |
| S. aureus Russell | 3.1 |
| S. aureus 1517 | 25 |
| S. pneumoniae CN 33 | 50 |
| S. pyogenes CN10 | 6.2 |
| E. cloacae T753 | 100 |
| E. coli E8 | 50 |
| E. coli E96 | 50 |
| K. aerogenes T767 | 50 |
| S. aureus MB9 | 25 |

MIC DATA (Broth) for sodium (5RS,6RS)-4,4-dimethyl-6-[(1SR)-1-hydroxyethyl]-3-(pyrimidin-2-ylsulphinyl)-7-oxo-1-azabicyclo[3.2.0]hept-2-ene-Z-carboxylate (27)

| Organism | MIC (μg/ml) |
|---|---|
| C. freundii E8 | 25 |
| E. cloacae N1 [T753] | 25 |
| E. coli 0111 [E8] | 25 |
| E. coli JT39 [E96] | 50 |
| E. coli ESS | 1.6 |
| K. aerogenes A | 50 |
| P. mirabilis 977 | 25 |
| P. morganii 1580 | 25 |
| P. rettgeri WM16 | 25 |
| P. vulgaris W091 | 6.2 |
| S. typhimurium CT10 | 12.5 |
| Serratia marcescens US32 | 100 |
| S. sonnei MB11 1967 | 50 |
| B. subtilis A | 0.8 |
| S. aureus Oxford | 0.8 |
| S. aureus Russell | 1.6 |
| S. aureus 1517 | 25 |
| E. coli JT410 C4 | 25 |
| E. coli JT 2074 | 25 |
| K. aeogenes BA 9J R+ | 25 |

Tissue Stability Data for sodium (5RS,6RS)-3-[(Z)-2-carbamoylethenylthio]-4,4-dimethyl-6-[(1SR)-1-hydroxyethyl]-7-oxo-1-azabicyclo[3.2.0]hept-2-en-2-carboxylate (32)

## Tissue Stability Values

A preparation of 2% mouse kidney homogenate was mixed with equal volumes of a 200 µg/ml solution of the antibiotic to be tested. The stability of the antibiotic at 37°C was studied by removal of aliquots and subjecting them to high pressure liquid chromatographic analysis. The values are given in figures relative to that obtained for MM13902 which is taken as a standard (1.0). Values greater than 1.0 reflect the fold increase in stability relative to MM13902. (For the structure of MM13902 see UK Patent No: 1489235).

Table 1

| Compound | Relative Stability |
|---|---|
| MM13902 | 1.4 |
| Example 26 (e32) | 10.8 |

In a comparable test on human kidney homogenate the values were 1.1 for MM13902 and 6.9 for the compound of Example 26.

CLAIMS:

1. A compound of the formula (I):

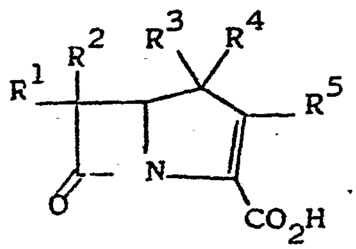

(I)

or a pharmaceutically acceptable salt or a pharmaceutically acceptable ester thereof wherein $R^1$ and $R^2$ each represent a hydrogen atom or an organic group bonded via a carbon atom to the azabicycloheptene ring, $R^3$ and $R^4$ each represent an organic group bonded via a carbon atom to the azabicycloheptene ring, or $R^3$ and $R^4$ are joined so as to form together with the carbon atom to which they are attached an optionally substituted $C_{3-7}$ cycloalkyl or optionally substituted heterocyclyl ring, and $R^5$ is a hydrogen atom, a group SH, or a group $S(O)_nR^6$ wherein n is zero or one and $R^6$ is an organic group bonded via a carbon atom to the sulphur atom, excluding benzyl 4,4-dimethyl-7-oxo-1-azabicyclo[3.2.0]hept-2-ene-2-carboxylate.

2. A compound as claimed in claim 1 wherein $R^5$ is a group $-S(O)_nR^6$ wherein n and $R^6$ are as defined in claim 1.

3. A compound as claimed in claim 2 wherein n is one.

4. A compound as claimed in any of claims 1 to 3 wherein $R^6$ is an optionally substituted pyrimidinyl group.

5. A compound as claimed in any of claims 1 to 3 wherein $R^6$ is $C_{1-6}$ alkanoylaminoethyl.

6. A compound as claimed in any of claims 1 to 3 wherein $R^6$ is vinyl optionally substituted by a group selected from carbamoyl, carboxy and salts and esters thereof, and $C_{1-6}$ alkanoylamino.

7. A compound as claimed in claim 6 wherein $R^6$ is acetamidovinyl.

8. A compound as claimed in claim 6 wherein $R^6$ is aminoethoxycarbonylvinyl.

9. A compound as claimed in any of claims 1 to 8 wherein $R^1$ is a hydrogen atom.

10. A compound as claimed in any of claims 1 to 9 wherein $R^2$ is a hydrogen atom.

11. A compound as claimed in any of claims 1 to 10 wherein one of $R^1$ and $R^2$ in hydrogen and the other is $\alpha$-sulphonato-oxyethyl.

12. A compound as claimed in any of claims 1 to 10 wherein one of $R^1$ and $R^2$ is hydrogen and the other is a $-C(CH_3)_2OH$, $-CH(CH_3)OH$, $-CH(C_2H_5)OH$ or $-CH(CH_3)_2$ group.

13. A compound as claimed in any of claims 1 to 12 wherein $R^3$ and $R^4$ are joined so as to form with the carbon to which they are attached a cyclopropyl ring.

14. A compound as claimed in any of claims 1 to 12 wherein $R^3$ and $R^4$ are independently selected from $C_{1-6}$ alkyl.

15. A compound as claimed in claim 14 wherein $R^3$ and $R^4$ are both methyl.

16. A compound as claimed in claim 1 selected from:

Sodium 4,4-dimethyl-3-ethylthio-7-oxo-1-azabicyclo [3.2.0]hept-2-en-2-carboxylate;
Sodium (5RS,6RS)4,4-dimethyl-6-[(1SR)-1-hydroxyethyl]-3-(pyrimidin-2-ylthio)-7-oxo-1-azabicyclo [3.2.0]hept-2-en-2-carboxylate,
Sodium (5RS,6RS)4,4-dimethyl-6-[(1SR)-1-hydroxyethyl]-3-(pyrimidin-2-ylsulphinyl)-7-oxo-1-azabicyclo[3.2.0]hept-2-en-2-carboxylate,
Sodium (5RS,6RS)-3-[(Z)-2-carbamoylethenylthio]-4,4-dimethyl-6-[(1SR)-1-hydroxyethyl]-7-oxo-1-azabicyclo[3.2.0]hept-2-en-2-carboxylate,
Sodium (5RS,6RS)-3-[(Z)-2-acetamidoethenylthio]-4,4-dimethyl-6-[(1SR)-1-hydroxyethyl]-7-oxo-1-azabicyclo[3.2.0]hept-2-en-2-carboxylate,
Sodium (5RS,6RS)-6-[(1SR)-1-hydroxyethyl]-3-(pyrimidin-2-ylthio)-7-oxospiro[[1]azabicyclo [3.2.0] hept[2]ene-4,1'-cyclopropane]-2-carboxylate,
Sodium (5RS,6RS)-6-[(1SR)-1-hydroxyethyl]-3-(pyrimidin-2-ylsulphinyl)-7-oxospiro[[1]azabicyclo [3.2.0]hept[2]-ene-4,1'-cyclopropane]-2-carboxylate

17. A pharmaceutical composition comprising a compound as claimed in any of claims 1 to 16 and a pharmaceutically acceptable carrier.

18. A process for the preparation of a compound of the formula (VI):

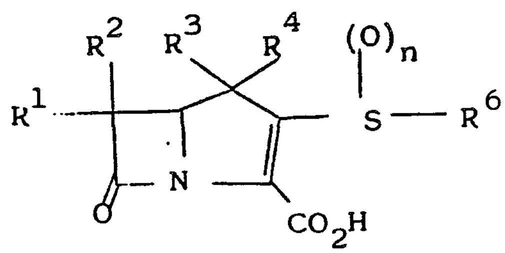

(VI)

or salt or ester thereof whether $R^1$, $R^2$, $R^3$, $R^4$, n and $R^6$ are as defined in claim 1; which comprises the elimination of the elements of HX from a compound of the formula (VII):

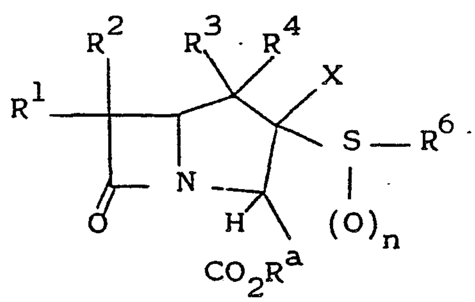

(VII)

wherein X is a halo group and $R^a$ is a carboxy-blocking group; and thereafter if necessary:

i) removing any carboxy-blocking group,
ii) converting the product into a pharmaceuti cally acceptable salt or ester,
iii) oxidising the sulphur atom to afford a sulphoxide.
(iv) converting a group $-SO-R^6$ to a group $-S-R^6$ wherein the groups $R^6$ are different.

19. A process for the preparation of a compound of the formula (XI):

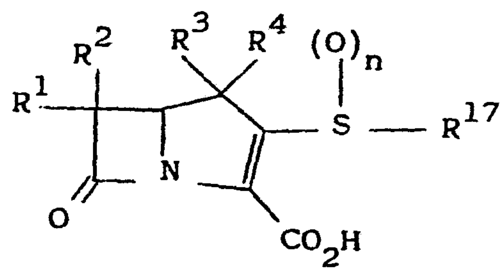

(XI)

or salt or ester thereof, wherein $R^1$, $R^2$, $R^3$, $R^4$ and n are as defined in claim 1 and $R^{17}$ is an organic radical bonded to the sulphur atom <u>via</u> the carbon atom, said radical having an unsaturated moiety located adjacent to the sulphur atom; which process comprises the elimination of the elements of $R^{14}R^{15}R^{16}P=0$ from an ester of a compound of the formula (XII):

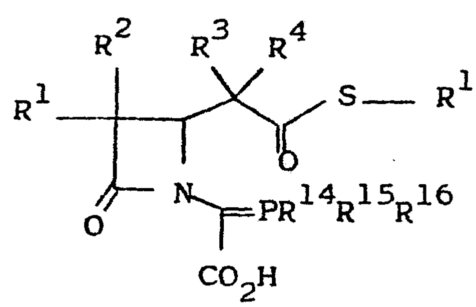

(XII)

wherein $R^{14}$, $R^{15}$ and $R^{16}$ are independently phenyl or methoxyphenyl, and thereafter if necessary:

i) converting the ester into an acid, salt or another ester,

ii) oxidising the sulphur atom to a sulphoxide group.

iii) converting a group $-SO-R^6$ to a group $-S-R^6$ wherein the groups $R^6$ are different.

0060077

European Patent Office

# EUROPEAN SEARCH REPORT

Application number

EP 82 30 1061

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| Y | EP-A-0 010 317 (MERCK)<br>*Claims; pages 120-135*<br>--- | 1-12, 17,19 | C 07 D 487/04<br>A 61 K 31/40 //<br>C 07 D 205/08<br>C 07 F 9/65<br>C 07 D 265/16<br>C 07 D 498/04<br>C 07 C 31/27<br>C 07 C 31/36<br>C 07 C 117/00<br>(C 07 D 487/04<br>C 07 D 209/00<br>C 07 D 205/00 ) |
| Y | EP-A-0 010 316 (MERCK)<br>*Claims; pages 90-95*<br>--- | 1-12, 17,19 | |
| P | EP-A-0 030 031 (MERCK)<br>*Claims*<br>--- | 1-19 | |
| P | EP-A-0 030 032 (MERCK)<br>*Claims*<br>--- | 1-19 | |
| P | EP-A-0 045 198 (TAKEDA)<br>*Claims*<br>----- | 1-12, 17,19 | TECHNICAL FIELDS SEARCHED (Int. Cl. 3)<br>C 07 D 487/00<br>A 61 K 31/00 |

The present search report has been drawn up for all claims

| Place of search | Date of completion of the search | Examiner |
|---|---|---|
| THE HAGUE | 03-05-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82